(19) Europäisches Patentamt

European Patent Office

Office européen des brevets

(11) **EP 2 547 810 B1**

(12) **EUROPEAN PATENT SPECIFICATION**

(45) Date of publication and mention
of the grant of the patent:
**06.03.2019 Bulletin 2019/10**

(21) Application number: **11709018.3**

(22) Date of filing: **16.03.2011**

(51) Int Cl.:
*D01F 1/02* (2006.01)     *D01F 4/00* (2006.01)
*D01F 6/68* (2006.01)     *C07K 14/425* (2006.01)
*C07K 14/435* (2006.01)

(86) International application number:
**PCT/EP2011/001307**

(87) International publication number:
**WO 2011/113592 (22.09.2011 Gazette 2011/38)**

(54) **METHOD FOR PRODUCTION OF POLYPEPTIDE CONTAINING FIBRES**

VERFAHREN ZUR HERSTELLUNG VON POLYMERHALTIGEN FASERN

PROCÉDÉ DE FABRICATION DE FIBRES CONTENANT UN POLYMÈRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.03.2010 PCT/EP2010/001694**

(43) Date of publication of application:
**23.01.2013 Bulletin 2013/04**

(73) Proprietor: **Amsilk GmbH**
**82152 Planegg/Martinsried (DE)**

(72) Inventors:
• **BAUSCH, Andreas**
**85716 Unterschleissheim (DE)**

• **RAMMENSEE, Sebastian**
**72070 Tübingen (DE)**

(74) Representative: **Geling, Andrea et al**
**ZSP Patentanwälte PartG mbB**
**Hansastraße 32**
**80686 München (DE)**

(56) References cited:
**EP-A1- 1 462 548     WO-A1-2006/081749
WO-A2-03/060099     WO-A2-2004/000915
WO-A2-2010/015419     US-A- 2 901 813
US-A1- 2009 259 010**

## Description

The present invention relates to a method of spinning a silk polypeptide fibre. It further relates to a silk polypeptide fibre obtainable by said method and to uses thereof. The invention also relates to products comprising said silk polypeptide fibre.

## BACKGROUND OF THE INVENTION

Conventional methods of polymer fibre formation have considerable shortcomings, regarding both methods and products. The methods usually have requirements too harsh for the involvement of biological material such as proteins or cells, regarding conditions in terms of temperature, pressure or the use of chemicals and solvents. The products are normally designed to feature superiority in one property, such as strength, elasticity or weight. However, combining all these properties has proved difficult.

Natural spider silk can assume different forms, depending on the gland it is produced in (Gosline et al., J. Exp. Biol. (202):3295, 1999). Its properties are remarkable: Its tensile strength can be superior to that of steel and equals that of aramid filaments, e.g. Kevlar. Spider silk can also be very ductile, being stretchable to up to about 300% of its length without tearing. Above all, it is lightweight.

Since native spider silk production is impractical due to the territorial and cannibalistic nature of spiders, scientific and commercial interest initiated the investigation of artificial spider silk manufacturing, with the goal of eventual industrial scale production. However, it has been difficult to find a commercially viable process to mass-produce spider silk. Artificial production has encountered problems in achieving both sufficient protein yield and quality thread assembly. The problem of the protein source has been addressed by the production of recombinant spider silk proteins by bacteria (Scheibel, Microb. Cell. Fact., (1):14, 2004). Still, fibre drawing from aqueous solutions of these proteins is not possible, since such a solution, by itself, does not exhibit sufficient elongational viscosity.

Spinning of fibres is a process in which deforming stresses in the direction of the fibre axis compete with surface tension of the spinning dope. For example, it is not possible to pull out a stable fluid filament of water due to the high surface tension of water.

This is illustrated by the Rayleigh instability, which causes a jet of water that runs out of a tap to break up into droplets. This effect is caused by the surface tension of water.

The Ohnesorge number Oh describes the ratio of viscous to surface tension forces.

$$Oh = \frac{\eta}{\sqrt{\rho \sigma R}}$$

Small Oh (Oh << 1) means, that a fluid filament will break up into small droplets driven by surface tension. High Oh means, that a fluid filament will remain stable (Edmond, Schofield et al. 2006). Effectively, this means that a spinning dope has to show a high Ohnesorge number. Obviously Oh can be increased by increasing the viscosity. In fluids with a high Trouton ratio, elongational viscosity will dominate the elongational flow processes, and therefore high Oh numbers can be reached in fluids which have a rather low shear viscosity $\eta$, but a high Trouton ratio Tr.

Recombinant spider silk protein can be produced by bacteria and its assembly has been studied in detail (Scheibel, Microb. Cell. Fact., (1):14, 2004; Huemmerich et al., Curr. Biol., (22):2070-4, 2004; Rammensee et al., PNAS, (105): 6590-6595, 2008). Artificial spinning dopes with low protein concentrations (10-20 mg/ml eADF3 and eADF4) do hot show Tr >>1. The surface tension is still much larger than the viscosity effects, so also Oh << 1, therefore, fibre formation from these low protein concentration solutions has not been possible. Only recombinant spider silk protein solutions with very high protein concentrations (~ 200 mg/ml) have allowed fibre formation (Exler et al., Angewandte Chemie-International Edition, (19):3559-3562, 2007). However, increasing the protein concentration increases the shear viscosity of the solution, and the processibility of a fluid with very high (> 1 Pa s) shear viscosity is rather poor.

The inventors of the present invention surprisingly found that only very low silk polypeptide concentrations are required for spinning a fibre with the method of the present invention. The use of very low silk polypeptide concentrations has the advantage, that the shear viscosity of the solution is not increased, which, in turn, improves the processability of the fluid.

Surprisingly, fibre formation from very low concentrated silk polypeptides such as spider silk polypeptides, under gentle conditions was facilitated by the inclusion of polyacrylamide (PAA), as elongational viscosity enhancer. Although spider silk dopes in nature have been described as containing very high percentages of spider silk protein, the present method surprisingly requires only very low concentrations of silk polypeptides beginning at concentrations as low as 0.15 mg/ml..

Further, the present method surprisingly requires only very low concentrations of the above-mentioned elongational viscosity enhancer. The low concentrations of PAA is sufficient to increase on the one hand side the elongational viscosity of the solution, but on the other hand side do not substantially increase the shear viscosity of the solution so that the generation of fibres is improved.

Thus, the use of very low concentrations of silk polypetides and/or very low concentrations of PAA in the method of the present invention facilitates the spinning of fibres from a spinning solution. Furthermore, the method has a low technical hurdle with fibres being drawn directly from the silk polypeptide solution at ambient or near ambient conditions.

## SUMMARY OF THE INVENTION

In a first aspect, the present invention relates to a method for fibre spinning from a spinning solution comprising the steps of:

(a) providing a spinning solution comprising (i) a silk polypeptide, (ii) polyacrylamide (PAA), and (iii) a solvent; and
(b) drawing a fibre from the spinning solution or a combination of extruding and drawing a fibre from the spinning solution, whereby a fibre is formed.

In a second aspect the present invention relates to a spinning solution for carrying out the method of the present invention, comprising (i) a silk polypeptide, (ii) polyacrylamide (PAA), and (iii) a solvent.

In a third aspect, the present invention relates to a fibre obtainable by the method of the first aspect and to its use.

In a fourth aspect, the present invention provides products comprising the fibre of the second aspect.

This summary of the invention does not necessarily describe all features of the invention.

## DETAILED DESCRIPTION OF THE INVENTION

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

In the following, the elements of the present invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step.

As used in this specification and the appended claims, the singular forms "a", "an", and "the" include plural referents, unless the content clearly dictates otherwise.

Residues in two or more polypeptides are said to "correspond" to each other if the residues occupy an analogous position in the polypeptide structures. It is well known in the art that analogous positions in two or more polypeptides can be determined by aligning the polypeptide sequences based on amino acid sequence or structural similarities. Such alignment tools are well known to the person skilled in the art and can be, for example, obtained on the World Wide Web, e.g., ClustalW (www.ebi.ac.uk/clustalw) or Align (http://www.ebi.ac.uk/emboss/align/index.html) using standard settings, preferably for Align EMBOSS: needle, Matrix: Blosum62, Gap Open 10.0, Gap Extend 0.5.

In a first aspect, the present invention relates to a method of spinning a fibre from a spinning solution comprising, essentially consisting of, or consisting of the steps of:

(a) providing a spinning solution comprising (i) a silk polypeptide, (ii) PAA, and (iii) a solvent; and
(b) drawing a fibre from the spinning solution or a combination of extruding and drawing a fibre from the spinning solution, whereby a fibre is formed.

In the context of the present invention, the term "polymer" refers to a molecule composed of repeating structural units typically connected by covalent chemical bonds. The polymer of the present invention is a biopolymer i.e. a silk polypeptide.

The term "biopolymer", as used herein, refers to a molecule containing monomeric units that are covalently bonded to form larger structures. Biopolymers can be classified in three main classes based on the differing monomeric units used and the structure of the biopolymers formed. The first class of biopolymers are polynucleotides which are polymers composed of nucleotide monomers, e.g. polymers composed of 10 or more nucleotide monomers. The second class of biopolymers are polypeptides which are polymers of amino acids. The third class of biopolymers are polysaccharides which are often linear bonded polymeric carbohydrate structures. The biopolymer of the present invention is at least a silk polypeptide.

Preferably, the biopolymers are biodegradable and more preferably also compostable. Biodegradable biopolymers are broken down into $CO_2$ and water by microorganisms. Biodegradable biopolymers which are also compostable can be put into an industrial composting process and will break down within months.

It is also preferred that the biopolymers are biocompatible. This means that the polymer is non-toxic, non-mutagenic and elicits no or only a minimal inflammatory reaction.

Thus, also fibres spun from a silk polypeptide with the method of the present invention can have the above mentioned advantageously characteristics, namely can be biodegradable, compostable and/or biocompatible. Particularly, as the amount of PAA is preferably very low in the spinning solution of the method of the present invention.

As already mentioned above, within a polypeptide, the repeating structural units are amino acids connected by covalent amide bonds (peptide bonds).

Unless otherwise indicated, the terms "polypeptide" and "protein" are used interchangeably herein and mean any peptide-linked chain of amino acids, regardless of length or post-translational modification.

Preferably, the polypeptide is a silk polypeptide comprising at least two identical repetitive units. Further polypeptides that can be used additionally are bovine serum albumin (BSA), zein or casein. These further polypeptide may also be collagen, fibronectin or keratin.

Bovine serum albumin (BSA), also known as "Fraction V", is a serum albumin protein. Zein is a prolamine protein found in maize. Casein (from Latin *caseus* "cheese") is the predominant phosphoprotein ($\alpha$S1, $\alpha$S2, $\beta$, $\kappa$) that accounts for nearly 80 % of proteins in cow milk and cheese.

In the context of the present invention, the term "silk polypeptide" refers to a silk polypeptide or protein (it is noted that, unless otherwise indicated, these two terms, as used herein, are interchangeable) that is expressed in a recombinant (e.g. microbial, yeast, plant, insect or mammalian) expression system, i.e. separated from its natural milieu in the spider gland (recombinant silk polypeptide or protein). Preferably, the "silk polypeptide" is isolated or purified. In particular, a "purified silk polypeptide" or an "isolated silk polypeptide" is free or substantially free of cellular material or other contaminating proteins from the cell or tissue source from which the protein is isolated or derived. The language "substantially free of cellular material" includes preparations of a silk polypeptide in which the silk polypeptide is separated from cellular components of the cells from which it is recombinantly produced. Thus, a silk polypeptide that is substantially free of cellular material includes preparations of silk polypeptides having less than about 30%, 20%, 10%, 5% or 1 % (by dry weight) of contaminating protein. When the silk polypeptide is expressed in cell culture, it is also free or substantially free of culture medium, i.e., the culture medium represents less than about 20%, 10%, 5% or 1 % of the volume of the polypeptide preparation.

A "silk polypeptide" as used in the context of the present invention further refers to a polypeptide with an amino acid sequence which comprises or consists of at least 50%, 60%, 65%, 70%, 75%, 80%, 85%, 90%, preferably at least 95% and most preferably 100% of multiple copies of one identical repetitive unit (e.g. $A_2$, $Q_6$, or $C_{16}$, wherein the items 2, 6, or 16 represent the number of repetitive units) or multiple copies of two or more different repetitive units (e.g. $(AQ)_{24}$, or $(AQ)_{12}$, $C_{16}$).

The terms "repetitive unit" and "repeat unit" can interchangeable be used in the context of the present invention.

In the context of the present invention, the term "silk polypeptide" also refers to a silk polypeptide that comprises or consists of at least two identical repetitive units which comprise or consists of identical copies of amino acid sequences of naturally-occurring silk polypeptides or of variations of amino acid sequences of naturally-occurring silk polypeptides or of combinations of both.

In the context of the present invention, a "repetitive unit" refers to a region which corresponds in amino acid sequence to a region that comprises or consists of at least one peptide motif (e.g. AAAAAA (SEQ ID NO: 13) or GPGQQ (SEQ ID NO: 4)) that repetitively occurs within a naturally occurring silk polypeptide (e.g. MaSpI, ADF-3, ADF-4, or Flag) (i.e. identical amino acid sequence) or to an amino acid sequence substantially similar thereto (i.e. variational amino acid sequence). In this regard "substantially similar" means a degree of amino acid identity of at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or even 99.9%, preferably over the whole length of the respective reference naturally occurring amino acid sequence. A "repetitive unit" having an amino acid sequence which is "substantially similar" to a corresponding amino acid sequence within a naturally occurring silk polypeptide (i.e. wild-type repetitive unit) is also similar with respect to its functional properties, e.g. a silk polypeptide comprising the "substantially similar repetitive unit" still has the ability to form a fibre. Preferably, it is still possible to draw a smooth and homogenous fibre from the silk polypeptide comprising the "substantially similar repetitive unit" at a speed of at least 0.1 cm/s, preferably 10 cm/s and more preferably 10 m/s. The skilled person can visually assess whether a fibre is still formed. The smooth and homogenous appearance of said fibre can be controlled using electronic-microscopy.

A "repetitive unit" having an amino acid sequence which is "identical" to the amino acid sequence of a naturally occurring silk polypeptide, for example, can be a portion of a silk polypeptide corresponding to one or more peptid motifs of MaSp I (SEQ ID NO: 43) MaSp II (SEQ ID NO: 44), ADF-3 (SEQ ID NO: 1) and/or ADF-4 (SEQ ID NO: 2). A "repetitive unit" having an amino acid sequence which is "substantially similar" to the amino acid sequence of a naturally occurring silk polypeptide, for example, can be a portion of a silk polypeptide corresponding to one or more peptide motifs of MaSpI (SEQ ID NO: 43) MaSpII (SEQ ID NO: 44), ADF-3 (SEQ ID NO: 1) and/or ADF-4 (SEQ ID NO: 2), but having one or more amino acid substitutions at specific amino acid positions.

The "repetitive unit" does not include the non-repetitive hydrophilic amino acid domains generally thought to be present at the carboxy and amino terminals of naturally occurring silk polypeptides.

A "repetitive unit" according to the present invention further refers to an amino acid sequence with a length of 3 to 200 amino acids, or 5 to 150 amino acids, preferably with a length of 10 to 100 amino acids, or 15 to 80 amino acids and more preferably with a length of 18 to 60, or 20 to 40 amino acids. For example, the repetitive unit according to the present invention can have a length of 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 105, 110, 115, 120, 125, 130, 135, 140, 145, 150, 155, 160, 165, 170, 175, 180, 185, 190, 195, or 200 amino acids. Most preferably, the repetitive unit according to the invention consists of 3, 4, 5, 6, 7, 8, 9, 10, 12, 15, 18, 20, 24, 27, 28, 30, 31, 32, 33, 34, 35, 36, 37, 38 or 39 amino acids.

The silk polypeptide as used in the method of the present invention preferably consists of between 6 to 1,500 amino acids, between 50 to 1,500 amino acids, or between 200 to 1,300 amino acids and most preferably between 250 to 1,200 amino acids, or between 500 to 1,000 amino acids.

Preferably, the silk polypeptide used in the method of the present invention comprises or consists of between 2 to 80 repetitive units, between 3 to 80 repetitive units, or between 4 to 60 repetitive units, more preferably between 8 to 48 repetitive units, or between 10 to 40 repetitive units and most preferably between 16 to 32 repetitive units. For example, the silk polypeptide used in the method of the present invention can comprise or consists of 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79 or 80 repetitive units. Most preferably, the silk polypeptide comprises 4, 8, 12, 16, 24, 32 or 48 repetitive units. As mentioned above, at least two of the repetitive units comprised in the silk polypeptide used in the method of the present invention are identical repetitive units. Thus, the silk polypeptide used in the method of the present invention may comprise multiple copies of one identical repetitive unit (e.g. $A_2$ or $C_{16}$, wherein the items 2 or 6 represent the number of repetitive units) or multiple copies of two or more different repetitive units (e.g. $(AQ)_{24}$ or $(QAQ)_8$). For example, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79 or 80 of the 80 repetitive units which may be comprised in the silk polypeptide used in the method of the present invention are identical repetitive units.

The silk polypeptide used in the method of the present invention can comprise or consist of an amino acid sequence of any silk polypeptide known to one skilled in the art. It is preferred that the silk polypeptide used in the method of the present invention comprises or consists of an amino acid sequence of an insect silk polypeptide, preferably of an arthropod polypeptide, or a spider silk polypeptide. The silk polypeptide used in the method of the present invention can also comprise or consist of an amino acid sequence of a mussel silk polypeptide.

It is preferred that the spider silk polypeptide comprises or consists of an amino acid sequence of a major ampullate gland polypeptide (MaSp), such as a dragline spider silk polypeptide, a minor ampullate gland polypeptide (MiSp), a flagelliform polypeptide, an aggregate spider silk polypeptide, an aciniform spider silk polypeptide or a pyriform spider silk polypeptide. Most preferably, the spider silk polypeptide comprises or consists of an amino acid sequence of a dragline spider silk polypeptide or a flagelliform spider silk polypeptide. It is generally preferred to select the amino acid sequence of the dragline polypeptide or flagelliform polypeptide of a dragline polypeptide or flagelliform polypeptide of orb-web spiders of Araneidae or Araneoids.

It is preferred that the insect silk polypeptide comprises or consists of an amino acid sequence of a silk polypeptide of Lepidoptera. More preferably, the insect silk polypeptide comprises or consists of an amino acid sequence of a silk polypeptide of Bombycidae, most preferably of *Bombyx mori*.

Preferably, the above mentioned silk polypeptides are recombinantly produced, i.e. are recombinant silk polypeptides. For example, the silk polypeptides used in the method of the present invention are recombinant spider silk polypeptides such as dragline spider silk polypeptides or flagelliform spider silk polypeptides, recombinant insect silk polypeptides, or recombinant mussel silk polypeptides.

The repetitive unit of the silk polypeptide used in the method of the present invention can comprise or consist of an amino acid sequence of any region that comprises or consists of at least one peptide motif that repetitively occurs within a naturally occurring silk polypeptide known to one skilled in the art. Preferably, the repetitive unit of the silk polypeptide used in the method of the present invention comprises or consists of an amino acid sequence of a region that comprises or consists of at least one peptide motif that repetitively occurs within an insect silk polypeptide, more preferably within an arthropod silk polypeptide or a spider silk polypeptide. The repetitive unit of the silk polypeptide used in the method of the present invention can also comprise or consist of an amino acid sequence of a region that comprises or consists of at least one peptide motif that repetitively occurs within a mussel silk polypeptide.

It is preferred that the spider silk repetitive unit comprises or consists of an amino acid sequence of a region that comprises or consists of at least one peptide motif that repetitively occurs within a naturally occurring major ampullate gland polypeptide (MaSp), such as a dragline spider silk polypeptide, a minor ampullate gland polypeptide (MiSp), a flagelliform polypeptide, an aggregate spider silk polypeptide, an aciniform spider silk polypeptide or a pyriform spider silk polypeptide. Most preferably, the repetitive unit comprises or consists of an amino acid sequence of a region that comprises or consists of at least one peptide motif that repetitively occurs within a naturally occurring dragline spider silk polypeptide or a flagelliform spider silk polypeptide.

It is preferred that the insect silk repetitive unit comprises or consists of an amino acid sequence of a region that comprises or consists of at least one peptide motif that repetitively occurs within a naturally occurring silk polypeptide of Lepidoptera. More preferably, the insect silk repetitive unit comprises or consists of an amino acid sequence of a region that comprises or consists of at least one peptide motif that repetitively occurs within a naturally occurring insect silk polypeptide of Bombycidae, most preferably of *Bombyx mori*.

The term "consensus sequence" as used in the context of the present invention refers to an amino acid sequence which contains amino acids which frequently occur in a certain position (e.g. "G") and wherein, other amino acids which are not further determined are replaced by the place holder "X".

Preferably, the silk polypeptide used in the method of the present invention comprises, essentially consists of, or consists of at least two identical repetitive units each comprising at least one, preferably one, consensus sequence selected from the group consisting of:

i) GPGXX (SEQ ID NO: 3), wherein X is any amino acid, preferably in each case independently selected from A, S, G, Y, P, N and Q;
ii) GGX, wherein X is any amino acid, preferably in each case independently selected from Y, P, R, S, A, T, N and Q, more preferably in each case independently selected from Y, P and Q; and
iii) $A_x$, wherein x is an integer from 5 to 10.

It is also preferred that the silk polypeptide used in the method of the present invention comprises or consists of at least two identical repetitive units each comprising at least one, preferably one amino acid sequence selected from the group consisting of: GGRPSDTYG (SEQ ID NO: 18) and GGRPSSSYG (SEQ ID NO: 19).

The iterated (peptide) motifs GPGXX (SEQ ID NO: 3) and GGX, i.e. glycine rich motifs, provide flexibility to the silk polypeptide and thus, to the thread formed from the silk protein containing said motifs. In detail, the iterated GPGXX (SEQ ID NO: 3) motif forms β-turn spiral structures, which imparts elasticity to the silk polypeptide. Major ampullate and flagelliform silks both have a GPGXX (SEQ ID NO: 3) motif. The iterated GGX motif is associated with a helical structure having three amino acids per turn and is found in most spider silks. The GGX motif may provide additional elastic properties to the silk. The iterated polyalanine $A_x$ (peptide) motif forms a crystalline β-sheet structure that provides strength to the silk polypeptide. (WO 03/057727). The GGRPSDTYG (SEQ ID NO: 18) and GGRPSSSYG (SEQ ID NO: 19) (peptide) motifs have been selected from Resilin (WO 08/155304). Resilin is an elastomeric protein found in most arthropods (*arthropoda*). It is located in specialised regions of the cuticle, providing low stiffness and high strength (Elvin et al., Nature (473): 999-1002, 2005).

Thus, in a preferred embodiment of the present invention, the silk polypeptide comprises or consists of repetitive units each comprising at least one (e.g. 1, 2, 3, 4, 5, 6, 7, 8, or 9), preferably one, amino acid sequence selected from the group consisting of GPGAS (SEQ ID NO: 5), GPGSG (SEQ ID NO: 6), GPGGY (SEQ ID NO: 7), GPGGP (SEQ ID NO: 8), GPGGA (SEQ ID NO: 9), GPGQQ (SEQ ID NO: 4), GPGGG (SEQ ID NO: 10), GPGQG (SEQ ID NO: 40), and GPGGS (SEQ ID NO: 11). In another preferred embodiment of the present invention, the silk polypeptide comprises or consists of repetitive units each comprising at least one (e.g. 1, 2, 3, 4, 5, 8, 7, or 8), preferably one, amino acid sequence selected from the group consisting of GGY, GGP, GGA, GGR, GGS, GGT, GGN, and GGQ. In a further preferred embodiment of the present invention, the silk polypeptide comprises or consists of repetitive units each comprising at least one (e.g. 1, 2, 3, 4, 5, or 6), preferably one, amino acid sequence selected from the group consisting of AAAAA (SEQ ID NO: 12), AAAAAA (SEQ ID NO: 13), AAAAAAA (SEQ ID NO: 14), AAAAAAAA (SEQ ID NO: 15), AAAAAAAAA (SEQ ID NO: 16), and AAAAAAAAAA (SEQ ID NO: 17).

In another preferred embodiment of the invention, the silk polypeptide comprises or consists of repetitive units each comprising at least one (e.g. 1, 2, 3, 4, 5, 6, 7, 8, 9, 10, 11, 12, 13, 14, 15, 16, 17, 18, 19, 20, 21, 22, 23, 24, or 25), preferably one, amino acid sequence selected from the group consisting of GPGAS (SEQ ID NO: 5), GPGSG (SEQ ID NO: 6), GPGGY (SEQ ID NO: 7), GPGGP (SEQ ID NO: 8), GPGGA (SEQ ID NO: 9), GPGQQ (SEQ ID NO: 4), GPGGG (SEQ ID NO: 10), GPGQG (SEQ ID NO: 40), GPGGS (SEQ ID NO: 11), GGY, GGP, GGA, GGR, GGS, GGT, GGN, GGQ, AAAAA (SEQ ID NO: 12), AAAAAA (SEQ ID NO: 13), AAAAAAA (SEQ ID NO: 14), AAAAAAAA (SEQ ID NO: 15), AAAAAAAAA (SEQ ID NO: 16), AAAAAAAAAA (SEQ ID NO: 17), GGRPSDTYG (SEQ ID NO: 18) and GGRPSSSYG (SEQ ID NO: 19).

Most preferably, the silk polypeptide used in the method of the present invention comprises, essentially consist of, or consists of repetitive units, which comprise or consist of

(i) GPGAS (SEQ ID NO: 5), AAAAAA (SEQ ID NO: 13), GGY, and GPGSG (SEQ ID NO: 6) as amino acid sequence, preferably in this order,
(ii) AAAAAAAA (SEQ ID NO: 15), GPGGY (SEQ ID NO: 7), GPGGY (SEQ ID NO: 7), and GPGGP (SEQ ID NO: 8) as amino acid sequence, preferably in this order,
(iii) GPGQQ (SEQ ID NO: 4), GPGQQ (SEQ ID NO: 4), GPGQQ (SEQ ID NO: 4) and GPGQQ (SEQ ID NO: 4) as amino acid sequence, preferably in this order,
(iv) GPGGA (SEQ ID NO: 9), GGP, GPGGA (SEQ ID NO: 9), GGP, GPGGA (SEQ ID NO: 9), and GGP as amino acid sequence, preferably in this order,
(v) AAAAAAAA (SEQ ID NO: 15), GPGQG (SEQ ID NO: 40), and GGR as amino acid sequence, preferably in this order,
(vi) AAAAAAAA (SEQ ID NO: 15), GPGGG (SEQ ID NO: 10), GGR, GGN, and GGR as amino acid sequence, preferably in this order,
(vii) GGA, GGA, GGA, GGS, GGA, and GGS as amino acid sequence, preferably in this order, and/or
(viii) GPGGA (SEQ ID NO: 9), GPGGY (SEQ ID NO: 7), GPGGS (SEQ ID NO: 11), GPGGY (SEQ ID NO: 7), GPGGS (SEQ ID NO: 11), and GPGGY (SEQ ID NO: 7) as amino acid sequence, preferably in this order.

It should be noted that at least two of the repetitive units comprised in the silk polypeptides mentioned above are identical repetitive units.

Preferably, the silk polypeptide used in the method of the present invention comprises, essentially consists of, or consists of between 2 to 80 repetitive units, between 3 to 80 repetitive units, or between 4 to 60 repetitive units, more preferably between 8 to 48 repetitive units, or between 10 to 40 repetitive units and most preferably between 16 to 32 repetitive units, i.e. 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79 or 80 repetitive units, each comprising at least one, preferably one, consensus sequence selected from the group consisting of:

i) GPGXX (SEQ ID NO: 3), wherein X is any amino acid, preferably in each case independently selected from A, S, G, Y, P, N and Q;
ii) GGX, wherein X is any amino acid, preferably in each case independently selected from Y, P, R, S, A, T, N and Q, more preferably in each case independently selected from Y, P and Q; and
iii) $A_x$, wherein x is an integer from 5 to 10.

As mentioned above, at least two of the repetitive units comprised in the silk polypeptide used in the method of the present invention are identical repetitive units.

It is also preferred that the silk polypeptide used in the method of the present invention comprises or consists of between 2 to 80 repetitive units, between 3 to 80 repetitive units, or between 4 to 60 repetitive units, more preferably between 8 to 48 repetitive units, or between 10 to 40 repetitive units and most preferably between 16 to 32 repetitive units, each comprising at least one, preferably one amino acid sequence selected from the group consisting of: GGRPSDTYG (SEQ ID NO: 18) and GGRPSSSYG (SEQ ID NO: 19).

Thus, the silk polypeptide used in the method of the present invention preferably comprises, essentially consists of, or consists of between 2 to 80 repetitive units, between 3 to 80 repetitive units, or between 4 to 60 repetitive units, more preferably between 8 to 48 repetitive units, or between 10 to 40 repetitive units and most preferably between 16 to 32 repetitive units, each comprising at least one (e.g. 1, 2, 3, 4, 5, 6, 7, 8, 9, 10, 11, 12, 13, 14, 15, 16, 17, 18, 19, 20, 21, 22, 23, 24, or 25), preferably one, amino acid sequence selected from the group consisting of GPGAS (SEQ ID NO: 5), GPGSG (SEQ ID NO: 6), GPGGY (SEQ ID NO: 7), GPGGP (SEQ ID NO: 8), GPGGA (SEQ ID NO: 9), GPGQQ (SEQ ID NO: 4), GPGQG (SEQ ID NO: 40), GPGGG (SEQ ID NO: 10), GPGGS (SEQ ID NO: 11), GGY, GGP, GGA, GGR, GGS, GGT, GGN, GGQ, AAAAA (SEQ ID NO: 12), AAAAAA (SEQ ID NO: 13), AAAAAAA (SEQ ID NO: 14), AAAAAAAA (SEQ ID NO: 15), AAAAAAAAA (SEQ ID NO: 16), AAAAAAAAAA (SEQ ID NO: 17), GGRPSDTYG (SEQ ID NO: 18) and GGRPSSSYG (SEQ ID NO: 19).

Most preferably, the silk polypeptide used in the method of the present invention comprises, essentially consists of, or consists of

(i) repetitive units which comprise or consist of GPGAS (SEQ ID NO: 5), AAAAAA (SEQ ID NO: 13), GGY, and GPGSG (SEQ ID NO: 6) as amino acid sequence, preferably in this order,
(ii) repetitive units which comprise or consist of AAAAAAAA (SEQ ID NO: 15), GPGGY (SEQ ID NO: 7), GPGGY (SEQ ID NO: 7), and GPGGP (SEQ ID NO: 8) as amino acid sequence, preferably in this order,
(iii) repetitive units which comprise or consist of GPGQQ (SEQ ID NO: 4), GPGQQ (SEQ ID NO: 4), GPGQQ (SEQ ID NO: 4) and GPGQQ (SEQ ID NO: 4) as amino acid sequence, preferably in this order,

(iv) repetitive units which comprise or consist of GPGGA (SEQ ID NO: 9), GGP, GPGGA (SEQ ID NO: 9), GGP, GPGGA (SEQ ID NO: 9), and GGP as amino acid sequence, preferably in this order,

(v) repetitive units which comprise or consist of AAAAAAAA (SEQ ID NO: 15), GPGQG (SEQ ID NO: 40), and GGR as amino acid sequence, preferably in this order,

(vi) repetitive units which comprise or consist of AAAAAAAA (SEQ ID NO: 15), GPGGG (SEQ ID NO: 10), GGR, GGN, and GGR as amino acid sequence, preferably in this order,

(vii) repetitive units which comprise or consist of GGA, GGA, GGA, GGS, GGA, and GGS as amino acid sequence, preferably in this order, and/or

(viii) repetitive units which comprise or consist of GPGGA (SEQ ID NO: 9), GPGGY (SEQ ID NO: 7), GPGGS (SEQ ID NO: 11), GPGGY (SEQ ID NO: 7), GPGGS (SEQ ID NO: 11), and GPGGY (SEQ ID NO: 7) as amino acid sequence, preferably in this order.

It should be noted that at least two of the repetitive units comprised in the silk polypeptides mentioned above are identical repetitive units.

Preferably, the silk polypeptide used in the method of the present invention comprises, essentially consist of, or consists of

(i) (GPGXX)$_n$ (SEQ ID NO: 3) as a repetitive unit, wherein X is any amino acid, preferably in each case independently selected from A, S, G, Y, P, N and Q and n is 2, 3, 4, 5, 6, 7, 8 or 9;

ii) (GGX)$_n$ as a repetitive unit, wherein X is any amino acid, preferably in each case independently selected from Y, P, R, S, A, T, N and Q, more preferably in each case independently selected from Y, P and Q, and n is 2, 3, 4, 5, 6, 7, or 8; and/or

iii) (A$_x$)$_n$ as a repetitive unit, wherein x is an integer from 5 to 10 and n is 2, 3, 4, 5, 6, 7, 8, 9, or 10.

As mentioned above, at least two of the repetitive units comprised in the silk polypeptides used in the method of the present invention are identical repetitive units.

It is preferred that the repetitive units are independently selected from module A (SEQ ID NO: 20), module C (SEQ ID NO: 21), module Q (SEQ ID NO: 22), module K (SEQ ID NO: 23), module sp (SEQ ID NO: 24), module S (SEQ ID NO: 25), module R (SEQ ID NO: 26), module X (SEQ ID NO: 27), or module Y (SEQ ID NO: 28), or variants thereof (i.e. module A variants, module C variants, module Q variants, module K variants, module sp variants, module S variants, module R variants, module X variants or module Y variants). The modules A (SEQ ID NO: 20) and Q (SEQ ID NO: 22) are based on the amino acid sequence of ADF-3 of the spider *Araneus diadematus.* Module C (SEQ ID NO: 21) is based on the amino acid sequence of ADF-4 of the spider *Araneus diadematus.* The modules K (SEQ ID NO: 23), sp (SEQ ID NO: 24), X (SEQ ID NO: 27) and Y (SEQ ID NO: 28) are based on the amino acid sequence of the flagelliform protein FLAG of the spider *Nephila clavipes* (WO 2006/008163). The modules S (SEQ ID NO: 25) and R (SEQ ID NO: 26) are based on Resilin (*Arthropoda*) (WO 2008/155304).

Thus, in a preferred embodiment of the present invention, the repetitive units of the silk polypeptide consist of module A: GPYGPGASAAAAAAGGYGPGSGQQ (SEQ ID NO: 20), module C: GSSAAAAAAAASGPGGYGPENQGPSGPGGYGP GGP (SEQ ID NO: 21), module Q: GPGQQGPGQQGPGQQGPGQQ (SEQ ID NO: 22), module K: GPGGAGGPYGPGGAGGPYGPGGAGGPY (SEQ ID NO: 23), module sp: GGTTIIEDLDITIDGADGPITISEELTI (SEQ ID NO: 24), module S: PGSSAAAAAAAASGPGQGQGQGQGQGGRPSDTYG (SEQ ID NO: 25), module R: SAAAAAAAAGPGGGNGGRPSDTYGAPGGGNGGRPSSSYG (SEQ ID NO: 26), module X: GGAGGAGGAGGSGGAGGS (SEQ ID NO: 27), or module Y: GPGGAGPGGYGPGGSGPGGYGPGGSGPGGY (SEQ ID NO: 28), or variants thereof.

Preferably, the silk polypeptide used in the method of the present invention comprises, essentially consists of, or consists of between 2 o 80 repetitive units, between 3 to 80 repetitive units, or between 4 to 60 repetitive units, more preferably between 8 to 48 repetitive units, or between 10 to 40 repetitive units and most preferably between 16 to 32 repetitive units, i.e. 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79 or 80 repetitive units, which are independently selected from module A (SEQ ID NO: 20), module C (SEQ ID NO: 21), module Q (SEQ ID NO: 22), module K (SEQ ID NO: 23), module sp (SEQ ID NO: 24), module S (SEQ ID NO: 25), module R (SEQ ID NO: 26), module X (SEQ ID NO: 27) or module Y (SEQ ID NO: 28), or variants thereof (i.e. module A variants, module C variants, module Q variants, module K variants, module sp variants, module S variants, module R variants, module X variants or module Y variants). It should be noted that at least two of the repetitive units comprised in the silk polypeptide used in the method of the present invention are identical repetitive units (modules).

Thus, it is preferred that the silk polypeptide used in the method of the present invention comprises, essentially consists of, or consists of (i) repetitive unit(s) consisting of module A and/or repetitive unit(s) consisting of module A variants, (ii) repetitive unit(s) consisting of module C and/or repetitive unit(s) consisting of module C variants, (iii) repetitive unit(s) consisting of module Q and/or repetitive unit(s) consisting of module Q variants, (iv) (a) repetitive unit(s) consisting of module A and repetitive unit(s) consisting of module Q, (b) repetitive unit(s) consisting of module A and repetitive unit(s) consisting of module Q variants, (c) repetitive unit(s) consisting of module A variants and repetitive unit(s) consisting of module Q, (d) repetitive unit(s) consisting of module A variants and repetitive unit(s) consisting of module Q variants, (v) (a) repetitive unit(s) consisting of module A and repetitive unit(s) consisting of module C, (b) repetitive unit(s) consisting of module A and repetitive unit(s) consisting of module C variants, (c) repetitive unit(s) consisting of module A variants and repetitive unit(s) consisting of module C, (d) repetitive unit(s) consisting of module A variants and repetitive unit(s) consisting of module C variants, (vi) (a) repetitive unit(s) consisting of module C and repetitive unit(s) consisting of module Q, (b) repetitive unit(s) consisting of module C and repetitive unit(s) consisting of module Q variants, (c) repetitive unit(s) consisting of module C variants and repetitive unit(s) consisting of module Q, (d) repetitive unit(s) consisting of module C variants and repetitive unit(s) consisting of module Q variants, or (vii) (a) repetitive unit(s) consisting of module A, repetitive unit(s) consisting of module Q and repetitive unit(s) consisting of module C, (b) repetitive unit(s) consisting of module A, repetitive unit(s) consisting of module Q and repetitive unit(s) consisting of module C variants, (c) repetitive unit(s) consisting of module A, repetitive unit(s) consisting of module Q variants and repetitive unit(s) consisting of module C, (d) repetitive unit(s) consisting of module A variants, repetitive unit(s) consisting of module Q and repetitive unit(s) consisting of module C, (e) repetitive unit(s) consisting of module A, repetitive unit(s) consisting of module Q variants and repetitive unit(s) consisting of module C variants, (f) repetitive unit(s) consisting of module A variants, repetitive unit(s) consisting of module Q variants and repetitive unit(s) consisting of module C, (g) repetitive unit(s) consisting of module A variants, repetitive unit(s) consisting of module Q and repetitive unit(s) consisting of module C variants, (h) repetitive unit(s) consisting of module A variants, repetitive unit(s) consisting of module Q variants and repetitive unit(s) consisting of module C variants.

The modules A, C, Q, K, sp, S, R, X or Y or variants thereof (i.e. module A variants, module C variants, module Q variants, module K variants, module sp variants, module S variants, module R variants, module X variants or module Y variants) can also be combined with each other in any combination and in any number of each, i.e. module (repetitive unit) A can be combined with module (repetitive unit) Q (i.e. combination AQ), module (repetitive unit) C can be combined with module (repetitive unit) Q (i.e. combination CQ), module (repetitive unit) Q can be combined with module (repetitive unit) A and with module (repetitive unit) Q (i.e. combination QAQ), module (repetitive unit) A can be combined with module (repetitive unit) A and with module (repetitive unit) Q (i.e. combination AAQ), etc., under the proviso that the silk polypeptide used in the method of the present invention comprises or consists of at least two repetitive units which are identical. For example, the silk polypeptide used in the method of the present invention can comprise or consist of $A_n$, $(AA)_n$, $(AQ)_n$, $(QA)_n$, $Q_n$, $(QQ)_n$, $(QAQ)_n$, $(AQA)_n$, $C_n$, $(CC)_n$, $(CCC)_n$, $(CQ)_n$, $(QC)_n$, $(QCQ)_n$, $(CQC)_n$, $(AA)_nQ_n$, $(QQ)_nA_n$, $(AAA)_nQ_n$, $(QQQ)_nA_n$, $(AQQ)_n$, $(QQA)_n$, $K_n$, $sp_n$, $S_n$, $R_n$, $X_n$, $Y_n$, $(Ksp)_n$, $(spK)_n$, $(XY)_n$, $(YX)_n$, $(XX)_n$, $(YY)_n$, $(XXX)_n$, $(YYY)_n$, $(AX)_n$, $(XA)_n$, $(CX)_n$, $(XC)_n$, $(QX)_n$, $(XQ)_n$, $(YQ)_n$, $(QY)_n$, $(SS)_n$, $(SR)_n$, $(RS)_n$, or $(RR)_n$, wherein n is at least 2, preferably 4, 8, 9, 10, 12, 16, 20, 24, or 32. In case that the silk polypeptide consists of $(AQ)_{12}$, it is noted that module (repetitive unit) A is 12 times present and module (repetitive unit) Q is also 12 times present in the silk polypeptide and that, thus, the silk polypeptide consists of 24 modules (repetitive units). The arrangement of the modules (repeat units) of a silk polypeptide consisting of $(AQ)_{12}$ is as follows: AQAQAQAQAQAQAQAQAQAQAQAQ. Further, in case that the silk polypeptide of the modules (repeat units) of a silk polypeptide consists of $(QAQ)_8$, it is noted that module (repeat unit) A is 8 times present and module (repetitive unit) Q is 16 times present in the silk polypeptide and that, thus, the silk polypeptide consists of 24 modules (repetitive units). The arrangement of the modules (repeat units) of a silk polypeptide consisting of $(QAQ)_8$ is as follows: QAQQAQQAQQAQQAQQAQQAQQAQ.

The silk polypeptide used in the method of the present invention can also comprise or consist of $(A^*Q)_n$, $(AQ^*)_n$, $(A^*Q^*)_n$, $(Q^*A)_n$, $(QA^*)_n$, $(Q^*A^*)_n$, $(QAQ^*)_n$, $(QA^*Q)_n$, $(Q^*AQ)_n$, $(QA^*Q^*)_n$, $(Q^*A^*Q)_n$, $(Q^*AQ^*)_n$, $(Q^*A^*Q^*)_n$, $(AQA^*)_n$, $(AQ^*A)_n$, $(A^*QA)_n$, $(AQ^*A^*)_n$, $(A^*Q^*A)_n$, $(A^*QA^*)_n$, $(A^*Q^*A^*)_n$, wherein n is at least 2, preferably 4, 8, 9, 10, 12, 16, 20, 24, or 32 and wherein * indicates a module variant, i.e. module A or Q variant.

The terms "combined with each other" or "concatenated with each other" may mean in the context of the present invention that the modules (repetitive units) are directly combined or concatenated with each other or may mean in the context of the present invention that the modules (repetitive units) are combined or concatenated with each other via one or more spacer amino acids. In preferred embodiments, the modules (repetitive units) comprised in the silk polypeptide are directly combined or concatenated with each other. In other preferred embodiments, the modules (repetitive units) comprised in the silk polypeptide are combined or concatenated with each other via 1 to 25 or 1 to 20 spacer amino acids, more preferably via 1 to 15 or 1 to 10 spacer amino acids, and most preferably, via 1 to 5 spacer amino acids, i.e. via 1, 2, 3, 4, 5, 6, 7, 8, 9, 10, 11, 12, 13, 14, 15, 16, 17, 18, 19, 20, 21, 22, 23, 24, or 25 spacer amino acids. Said spacer amino acids may be any amino acids naturally occurring in proteins. Preferably, said spacer amino acid is not proline. It is preferred that the spacer amino acid(s) contain(s) charged groups. Preferably, the spacer amino acid(s) containing charged groups is (are) independently selected from the group consisting of aspartate, glutamate, histidine, and lysine. Said spacer amino acids should be amino acids which do not negatively affect the ability of a silk polypeptide to coat, preferably to uniformly coat, the inert or naturally occurring material, such as kevlar or wool. Further, said spacer amino acids should be amino acids which do not cause steric hindrance, e.g. amino acids having a small size such as lysine and cysteine.

In more preferred embodiments, the silk polypeptide comprises modules which are directly combined with each other and modules which are combined with each other via 1 to 25 or 1 to 20 spacer amino acids, more preferably via 1 to 15 or 1 to 10 spacer amino acids, and most preferably, via 1 to 5 spacer amino acids, i.e. via 2, 3, 4, 5, 6, 7, 8, 9, 10, 11, 12, 13, 14, 15, 16, 17, 18, 19, 20, 21, 22, 23, 24, or 25 spacer amino acids.

A module A, C, Q, K, sp, S, R, X or Y variant differs from the reference (wild-type) module A, C, Q, K, sp, S, R, X or Y from which it is derived by up to 1, 2, 3, 4, 5, 6, 7, 8, 9, 10, 11, 12, 13, 14, or 15 amino acid changes in the amino acid sequence (i.e. substitutions, additions, insertions, deletions, N-terminal truncations and/or C-terminal truncations). Such a module variant can alternatively or additionally be characterised by a certain degree of sequence identity to the reference (wild-type) module from which it is derived. Thus, a module A, C, Q, K, sp, S, R, X or Y variant has a sequence identity of at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or even 99.9% to the respective reference (wild-type) modul A, C, Q, K, sp, S, R, X or Y. Preferably, the sequence identity is over a continuous stretch of at least 10, 15, 18, 20, 24, 27, 28, 30, 34, 35, or more amino acids, preferably over the whole length of the respective reference (wild-type) module A, C, Q, K, sp, S, R, X or Y.

It is particularly preferred that the sequence identity is at least 80% over the whole length, is at least 85% over the whole length, is at least 90% over the whole length, is at least 95% over the whole length, is at least 98% over the whole length, or is at least 99% over the whole length of the respective reference (wild-type) module A, C, Q, K, sp, S, R, X or Y. It is further particularly preferred that the sequence identity is at least 80% over a continuous stretch of at least 10, 15, 18, 20, 24, 28, or 30 amino acids, is at least 85% over a continuous stretch of at least 10, 15, 18, 20, 24, 28, or 30 amino acids, is at least 90% over a continuous stretch of at least 10, 15, 18, 20, 24, 28, or 30 amino acids, is at least 95% over a continuous stretch of at least 10, 15, 18, 20, 24, 28, or 30 amino acids, is at least 98% over a continuous stretch of at least 10, 15, 18, 20, 24, 28, or 30 amino acids, or is at least 99% over a continuous stretch of at least 10, 15, 18, 20, 24, 28, or 30 amino acids of the respective reference (wild-type) module A, C, Q, K, sp, S, R, X or Y.

A fragment (or deletion variant) of module A, C, Q, K, sp, S, R, X or Y has preferably a deletion of up to 1, 2, 3, 4, 5, 6, 7, 8, 9, 10, 11, 12, 13, 14, or 15 amino acids at its N-terminus and/or at its C-terminus. The deletion can also be internally.

Additionally, the module A, C, Q, K, sp, S, R, X or Y variant or fragment is only regarded as a module A, C, Q, K, sp, S, R, X or Y variant or fragment within the context of the present invention, if the modifications with respect to the amino acid sequence on which the variant or fragment is based do not negatively affect the ability of a silk polypeptide to form a fibre. Preferably, it is still possible to draw a smooth and homogenous fibre from the silk polypeptide comprising the module A, C, Q, K, sp, S, R, X or Y variant or fragment at a speed of at least 0.1 cm/s, preferably 10 cm/s and more preferably 10 m/s. The skilled person can visually assess whether a fibre is still formed. The smooth and homogenous appearance of said fibre can be controlled using electronic-microscopy.

Thus, in a preferred embodiment of the present invention the repetitive units are independently selected from module $A^C$ (SEQ ID NO: 29), module $A^K$ (SEQ ID NO: 30), module $C^C$ (SEQ ID NO: 31), module $C^{K1}$ (SEQ ID NO: 32), module $C^{K2}$ (SEQ ID NO: 33) or module $C^{KC}$ (SEQ ID NO: 34). The modules $A^C$ (SEQ ID NO: 29), $A^K$ (SEQ ID NO: 30), $C^C$ (SEQ ID NO: 31), $C^{K1}$ (SEQ ID NO: 32), $C^{K2}$ (SEQ ID NO: 33) and $C^{KC}$ (SEQ ID NO: 34) are variants of the module A which is based on the amino acid sequence of ADF-3 of the spider Araneus diadematus and of module C which is based on the amino acid sequence of ADF-4 of the spider Araneus diadematus (WO 2007/025719). In module $A^C$ (SEQ ID NO: 29) the amino acid S (serine) at position 21 has been replaced by the amino acid C (cysteine), in module $A^K$ (SEQ ID NO: 30) the amino acid S at position 21 has been replaced by the amino acid K (lysine), in module $C^C$ (SEQ ID NO: 31) the amino acid S at position 25 has been replaced by the amino acid 25 by C, in module $C^{K1}$ (SEQ ID NO: 32) the amino acid S at position 25 has been replaced by the amino acid K, in module $C^{K2}$ (SEQ ID NO: 33) the amino acid E (glutamate) at position 20 has been replaced by the amino acid K, and in module $C^{KC}$ (SEQ ID NO: 34) the amino acid E at position 20 has been replaced by the amino acid K and the amino acid S at position 25 has been replaced by the amino acid C (WO 2007/025719).

Preferably, the repetitive units in the silk polypeptide used in the method of the present invention consists of module $A^C$: GPYGPGASAAAAAAGGYGPGCGQQ (SEQ ID NO: 29), module $A^K$: GPYGPGASAAAAAAGGYGPGKGQQ (SEQ ID NO: 30), module $C^C$: GSSAAAAAAASGPGGYGPENQGPCGPG-GYGPGGP (SEQ ID NO: 31), module $C^{K1}$: GSSAAAAAAASGPGGYGPENQGPKGPGGYGPG-GP (SEQ ID NO: 32), module $C^{K2}$: GSSAAAAAAASG-PGGYGPKNQGPSGPGGYGPGGP (SEQ ID NO: 33), or module $C^{KC}$: GSSAAAAAAASGPGGYGPKNQG-PCGPGGYG PGGP (SEQ ID NO: 34).

It is also preferred that the silk polypeptide used in the method of the present invention comprises, essentially consists of, or consists of between 2 to 80 repetitive units, between 3 to 80 repetitive units, or between 4 to 60 repetitive units, preferably between 8 to 48 repetitive units, or between 10 to 40 repetitive units and most preferably between 16 to 32 repetitive units, i.e. 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79 or 80 repetitive units, which are independently selected from module $A^C$ (SEQ ID NO: 29), module $A^K$ (SEQ ID NO: 30), module $C^C$ (SEQ ID NO: 31), module $C^{K1}$ (SEQ ID NO: 32), module $C^{K2}$ (SEQ ID NO: 33) or module $C^{KC}$ (SEQ ID NO: 34). It should be noted that at least two of the repetitive units comprised in the silk polypeptide used in the method of the present invention are identical repetitive units (modules).

For example, the silk polypeptide used in the method of the present invention can comprises or consists of the modules $C^C_4$, $C^C_8$, $C^C_{16}$, $C^C_{32}$, $A^C_5$, $A^C_8$, or $A^C_{10}$.

The modules $A^K$, $C^C$, $C^{K1}$, $C^{K2}$ and $C^{KC}$ can also be combined with each other, i.e. module (repetitive unit) $A^K$ can be combined with module (repetitive unit) $C^C$ (i.e. combination $A^K C^C$), module (repetitive unit) $C^{K1}$ can be combined with module (repetitive unit) $C^{K2}$ and with module (repetitive unit) $C^{KC}$ (i.e. combination $C^{K1}C^{K2}C^{KC}$), etc., under the proviso that the silk polypeptide used in the method of the present invention comprises or consists of at least two repetitive units which are identical. Thus, the silk polypeptide used in the method of the present invention can also comprise or consist of the modules $(A^K)_n$, $(C^C)_n$, $(C^{K1})_n$, $(C^{K2})_n$, $(C^{KC})_n$, $(A^KA^C)_n$, $(C^CC^C)_n$, $(C^{K1}C^{K2})_n$, $(C^{K2}C^{K1})_n$, $(C^{K1}C^{K2}C^{K1})_n$, $(C^{K2}C^{K1}C^{K2})_n$, $(C^{K1}C^{K2}C^{KC})_n$, $(C^{KC}C^{K2}C^{KC})_n$, or $(C^{KC}C^{K2}C^{K1})_n$, wherein n is at least 2, preferably 4, 5, 6, 7, 8, 10, 12, 16, or 20. The term "combined with each other" is defined above.

It is further preferred that the silk polypeptide used in the method of the present invention comprises, essentially consists of, or consists of between 2 to 80 repetitive units, between 3 to 80 repetitive units or between 4 to 60 repetitive units, preferably between 8 to 48 repetitive units, or between 10 to 40 repetitive units and most preferably between 16 to 32 repetitive units, i.e. 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79 or 80 repetitive units, which are independently selected from module A (SEQ ID NO: 20) or variants thereof, module C (SEQ ID NO: 21) or variants thereof, module Q (SEQ ID NO: 22) or variants thereof, module K (SEQ ID NO: 23) or variants thereof, module sp (SEQ ID NO: 24) or variants thereof, module S (SEQ ID NO: 25) or variants thereof, module R (SEQ ID NO: 26) or variants thereof, module X (SEQ ID NO: 27) or variants thereof, module Y (SEQ ID NO: 28) or variants thereof, module $A^C$ (SEQ ID NO: 29), module $A^K$ (SEQ ID NO: 30), module $C^C$ (SEQ ID NO: 31), module $C^{K1}$ (SEQ ID NO: 32), module $C^{K2}$ (SEQ ID NO: 33) or module $C^{KC}$ (SEQ ID NO: 34). Again, it should be noted that at least two of the repetitive units comprised in the silk polypeptide used in the method of the present invention are identical repetitive units (modules).

The modules $A^K$, $C^C$, $C^{K1}$, $C^{K2}$ and $C^{KC}$ can also be combined with the modules A, C, Q, K, sp, S, R, X or Y, i.e. module (repetitive unit) $A^K$ can be combined with module (repetitive unit) C (i.e. combination $A^KC$), or module (repetitive unit) $C^C$ can be combined with module (repetitive unit) C (i.e. combination $C^CC$), etc., under the proviso that the silk polypeptide used in the method of the present invention comprises or consists of at least two repetitive units which are identical. Thus, the silk polypeptide used in the method of the present invention can also comprise or consist of the modules $(AQA^K)_n$, $(QA^K)_n$, $(QA^KQ)_n$, $(A^KQA)_n$, $(A^KQA^K)_n$, $(CC^C)_n$, $(CC^CC)_n$, $(C^CC^CC)_n$, $(CC^CC^C)_n$, $(C^CQ)_n$, $(QC^C)_n$, $(QC^CQ)_n$, $(C^CQC)_n$, $(CQC^C)_n$, $(C^CQC^C)_n$, $(CC^{K1})_n$, $(C^{K1}C)_n$, $(C^{K1}CC)_n$, $(CC^{K1}C)_n$, $(C^{KC}C^{KC}C)_n$, $(CC^{KC}C^{KC})_n$, $(C^{KC}Q)_n$, $(QC^{KC})_n$, $(QC^{KC}Q)_n$, $(A^{KC}\ ^{K1}Q)_n$, $(QC^{K2}A^K)_n$, or $(C^{K1}C^{K2}C)_n$, wherein n is at least 2, preferably 4, 5, 6, 7, 8, 10, 12, 16, or 20. The term "combined with each other" is defined above.

For example, the silk polypeptide used in the method of the present invention comprises or consists of the modules $C_{16}C^C$, $C^CC_{16}$, $C_8C^CC_8$, $C_8C^CC_8$, $C^CC_8C_8$, $C_4C^C_8C_4$, $C^C_4C_8C^C_4$, $C^C(AQ)_{24}$, or $(AQ)_{24}C^C$.

The silk polypeptide used in the method of the present invention can further comprise at least one non-repetitive (NR) unit, i.e. 1, 2, 3, 4, 5, 6, or more NR units, preferably one NR unit. In the context of the present invention, the term "non-repetitive (NR) unit" refers to a region of amino acids present in a naturally occurring silk polypeptide that displays no obvious repetition pattern (non-repetitive unit or NR unit). Preferably, the amino acid sequence of the non-repetitive unit corresponds to a non-repetitive amino acid sequence of naturally occurring dragline polypeptides, preferably of ADF-3 (SEQ ID NO: 1) or ADF-4 (SEQ ID NO: 2), or to an amino acid sequence substantially similar thereto.

It is particularly preferred that the amino acid sequence of the non-repetitive unit corresponds to a non-repetitive carboxy terminal amino acid sequence of naturally occurring dragline polypeptides, preferably of ADF-3 (SEQ ID NO: 1) or ADF-4 (SEQ ID NO: 2), or to an amino acid sequence substantially similar thereto. More preferably, the amino acid sequence of the non-repetitive unit corresponds to a non-repetitive carboxy terminal amino acid sequence of ADF-3 (SEQ ID NO: 1) which comprises amino acids 513 through 636, or of ADF-4 (SEQ ID NO: 2) which comprises amino acids 302 through 410, or to an amino acid sequence substantially similar thereto.

In this regard "substantially similar" means a degree of amino acid identity of at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or even 99.9%, preferably over 20, 30, 40, 50, 60, 70, 80 or more amino acids, more preferably over the whole length of the respective reference non-repetitive (carboxy terminal) amino acid sequence of naturally occurring dragline polypeptides, preferably of ADF-3 (SEQ ID NO: 1) or ADF-4 (SEQ ID NO: 2).

A "non-repetitive unit" having an amino acid sequence which is "substantially similar" to a corresponding non-repetitive (carboxy terminal) amino acid sequence within a naturally occurring dragline polypeptide (i.e. wild-type non-repetitive (carboxy terminal) unit), preferably within ADF-3 (SEQ ID NO: 1) or ADF-4 (SEQ ID NO: 2), is also similar with respect to its functional properties, e.g. a silk polypeptide comprising the "substantially similar non-repetitive unit" still has the ability to form a fibre. Preferably, it is still possible to draw a smooth and homogenous fibre from the silk polypeptide comprising the "substantially similar non-repetitive unit" at a speed of at least 0.1 cm/s, preferably 10 cm/s and more preferably 10 m/s. The skilled person can visually assess whether a fibre is still formed. The smooth and homogenous appearance of said fibre can be controlled using electronic-microscopy.

Most preferably, the non-repetitive (NR) unit is NR3 (SEQ ID NO: 41) or variants thereof, or NR4 (SEQ ID NO: 42) or variants thereof. The NR3 (SEQ ID NO: 41) unit is based on the amino acid sequence of ADF-3 of the spider *Araneus diadematus* and the NR4 (SEQ ID NO: 42) unit is based on the amino acid sequence of ADF-4 of the spider *Araneus diadematus* (WO 2006/008163).

A NR3 or NR4 unit variant differs from the reference NR3 (SEQ ID NO: 41) or NR4 (SEQ ID NO: 42) unit from which it is derived by up to 1, 2, 3, 4, 5, 6, 7, 8, 9, 10, 11, 12, 13, 14, 15, 16, 17, 18, 19, 20, 25, or 30 amino acid changes in the amino acid sequence (i.e. exchanges, insertions, deletions, N-terminal truncations and/or C-terminal truncations). Such a NR3 or NR4 unit variant can alternatively or additionally be characterised by a certain degree of sequence identity to the reference NR3 or NR4 unit from which it is derived. Thus, a NR3 or NR4 unit variant has a sequence identity of at least 50%, 55%, 60%, 65%, 70%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or even 99.9% to the respective reference NR3 or NR4 unit. Preferably, the sequence identity is over a continuous stretch of at least 10, 20, 30, 40, 50, 60, 70, 80, 90, or more amino acids, preferably over the whole length of the respective reference NR3 or NR4 unit.

It is particularly preferred that the sequence identity is at least 80% over the whole length, is at least 85% over the whole length, is at least 90% over the whole length, is at least 95% over the whole length, is at least 98% over the whole length, or is at least 99% over the whole length of the respective reference NR3 or NR4 unit. It is further particularly preferred that the sequence identity is at least 80% over a continuous stretch of at least 20, 30, 40, 50, 60, 70, or 80 amino acids, is at least 85% over a continuous stretch of at least 20, 30, 40, 50, 60, 70, or 80 amino acids, is at least 90% over a continuous stretch of at least 20, 30, 40, 50, 60, 70, or 80 amino acids, is at least 95% over a continuous stretch of at least 20, 30, 40, 50, 60, 70, or 80 amino acids, is at least 98% over a continuous stretch of at least 20, 30, 40, 50, 60, 70, or 80 amino acids, or is at least 99% over a continuous stretch of at least 20, 30, 40, 50, 60, 70, or 80 amino acids of the respective reference NR3 or NR4 unit.

A fragment (or deletion variant) of a NR3 or NR4 unit has preferably a deletion of up to 1, 2, 3, 4, 5, 6, 7, 8, 9, 10, 11, 12, 13, 14, 15, 16, 17, 18, 19, 20, 21, 22, 23, 24, 25, 30, 35, 40, 45, 50, 55, or 60 amino acids at its N-terminus and/or at its C-terminus. The deletion can also be internally.

Additionally, the NR3 or NR4 unit variant or fragment is only regarded as a NR3 or NR4 unit variant or fragment within the context of the present invention, if the modifications with respect to the amino acid sequence on which the variant or fragment is based do not negatively affect the ability of a silk polypeptide to form a fibre. Preferably, it is still possible to draw a smooth and homogenous fibre from the silk polypeptide comprising the NR3 or NR4 unit variant or fragment at a speed of at least 0.1 cm/s, preferably 10 cm/s, 20 cm/s, 50 cm/s, 75 cm/ s, 1 m/s, 2 m/s, 3 m/s, 4 m/s, 5 m/s and more preferably 10 m/s. The skilled person can visually assess whether a fibre is still formed. The smooth and homogenous appearance of said fibre can be controlled using electronic-microscopy.

Alternatively, it can be tested whether the NR3 or NR4 unit variant or fragment still enables the polymerization and/or increases the solubility of a silk polypeptide wherein it is comprised. The skilled person can readily assess whether a silk polypeptide comprising a NR3 or NR4 unit variant or fragment has the above mentioned functional properties like a silk polypeptide comprising the respective reference NR3 or NR4 unit. Suitable assays are well known to the person skilled in the art. For example, the polymerization of silk polypeptides comprising a NR3 or NR4 unit variant or fragment and the polymerization of silk polypeptides comprising the respective reference NR3 or NR4 unit can easily be visualized via native gel electrophoresis. The solubility of a silk polypeptide comprising a NR3 or NR4 unit variant or fragment and the solubility of a silk polypeptide comprising the respective reference NR3 or NR4 unit can simply be tested via saturation of said silk polypeptides in an aqueous solution. The results can finally be compared with each other.

Preferably, the silk polypeptide used in the method of the present invention is selected from the group consisting of ADF-3 (SEQ ID NO: 1) or variants thereof, ADF-4 (SEQ ID NO: 2) or variants thereof, MaSp I (SEQ ID NO: 43) or variants thereof, MaSp II (SEQ ID NO: 44) or variants thereof, $(C)_m$, $(C)_m NR_z$, $NR_z(C)_m$, $(AQ)_n$, $(AQ)_n NR_z$, $NR_z(AQ)_n$, $(QAQ)_o$, $NR_z(QAQ)_o$, $(QAQ)_o NR_z$, $Y_p$, $X_p$, and $K_p$, wherein m is an integer of 8 to 48 (i.e. 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, or 48), n is an integer of 4 to 24 (i.e. 4, 5, 6, 7, 8, 9, 10, 11, 12, 13, 14, 15, 16, 17, 18, 19, 20, 21, 22, 23, or 24), o is an integer of 2 to 20 (i.e. 2, 3, 4, 5, 6, 7, 8, 9, 10, 11, 12, 13, 14, 15, 16, 17, 18, 19, or 20), p is an integer of 8 to 16 (i.e. 8, 9, 10, 11, 12, 13, 14, 15, or 16) and z is an integer of 1 to 3 (i.e. 1, 2, or 3), preferably 1, and NR stands for a non-repetitive unit. The above mentioned formulas are defined by one of the following: In the formula

(i) $(C)_m$, a "m" number of C modules, namely 8 to 48 C modules, represented by the amino acid sequence according to SEQ ID NO: 21, are combined with each other ,

(ii) $(C)_m NR_z$, a "m" number of C modules, namely 8 to 48 C modules, represented by the amino acid sequence according to SEQ ID NO: 21, are combined with each other, wherein said C modules are further combined with a "z" number of non-repetitive (NR) units, namely 1 to 3 non-repetitive (NR) units, e.g. the non-repetitive (NR) units NR3 represented by the amino acid sequence according to SEQ ID NO: 41 or NR4 represented by the amino acid sequence according to SEQ ID NO: 42,

(iii) $NR_z(C)_m$, a "z" number of non-repetitive (NR) units, namely 1 to 3 non-repetitive (NR) units, e.g. the non-repetitive (NR) units NR3 represented by the amino acid sequence according to SEQ ID NO: 41 or NR4 represented by the amino acid sequence according to SEQ ID NO: 42, is present (z = 1) or are combined with each other (z = 2 or 3), wherein said non-repetitive (NR) unit(s) is (are) further combined with a "m" number of C modules, namely 8 to 48 C modules, represented by the amino acid sequence according to SEQ ID NO: 21,

(iv) $(AQ)_n$, a "n" number of A and Q module combinations, namely 6 to 24 A and Q module combinations, wherein module A is represented by the amino acid sequence according to SEQ ID NO: 20 and module Q is represented by the amino acid sequence according to SEQ ID NO: 22, are combined with each other,

(v) $(AQ)_n NR_z$, a "n" number of A and Q module combinations, namely 6 to 24 A and Q module combinations, wherein module A is represented by the amino acid sequence according to SEQ ID NO: 20 and module Q is represented by the amino acid sequence according to SEQ ID NO: 22, are combined with each other, and wherein said A and Q module combinations are further combined with a "z" number of non-repetitive (NR) units, namely 1 to 3 non-repetitive (NR) units, e.g. the non-repetitive (NR) units NR3 represented by the amino acid sequence according to SEQ ID NO: 41 or NR4 represented by the amino acid sequence according to SEQ ID NO: 42,

(vi) $NR_z(AQ)_n$, a "z" number of non-repetitive (NR) units, namely 1 to 3 non-repetitive (NR) units, e.g. the non-repetitive (NR) units NR3 represented by the amino acid sequence according to SEQ ID NO: 41 or NR4 represented by the amino acid sequence according to SEQ ID NO: 42, is present (z = 1) or are combined with each other (z = 2 or 3), wherein said non-repetitive (NR) unit(s) is (are) further combined with a "n" number of A and Q module combinations, namely 6 to 24 A and Q module combinations, wherein module A is represented by the amino acid sequence according to SEQ ID NO: 20 and module Q is represented by the amino acid sequence according to SEQ ID NO: 22,

(vii) $(QAQ)_o$, a "o" number of Q, A and Q module combinations, namely 8 to 16 Q, A and Q module combinations, wherein module Q is represented by an amino acid sequence according to SEQ ID NO: 22 and module A is represented by the amino acid sequence according to SEQ ID NO: 20, are combined with each other,

(viii) $(QAQ)_o NR_z$, a "o" number of Q, A and Q module combinations, namely 8 to 16 Q, A and Q module combinations, wherein module Q is represented by an amino acid sequence according to SEQ ID NO: 22 and module A is represented by the amino acid sequence according to SEQ ID NO: 20, are combined with each other, and wherein said Q, A and Q module combinations are further combined with a "z" number of non-repetitive (NR) units, namely 1 to 3 non-repetitive (NR) units, e.g. the non-repetitive (NR) units NR3 represented by the amino acid sequence according to SEQ ID NO: 41 or NR4 represented by the amino acid sequence according to SEQ ID NO: 42,

(ix) $NR_z(QAQ)_o$, a "z" number of non-repetitive (NR) units, namely 1 to 3 non-repetitive (NR) units, e.g. the non-repetitive (NR) units NR3 represented by the amino acid sequence according to SEQ ID NO: 41 or NR4 represented by the amino acid sequence according to SEQ ID NO: 42, is present (z = 1) or are combined with each other (z = 2 or 3), wherein said non-repetitive (NR) unit(s) is (are) further combined with a "o" number of Q, A and Q module combinations, namely 8 to 16 Q, A and Q module combinations, wherein module Q is represented by an amino acid sequence according to SEQ ID NO: 22 and module A is represented by the amino acid sequence according to SEQ ID NO: 20,

(x) $Y_p$, a "p" number of Y modules, namely 8 to 16 Y modules, represented by the amino acid sequence according to SEQ ID NO: 28, are combined with each other,

(xi) $X_p$, a "p" number of X modules, namely 8 to 16 X modules, represented by the amino acid sequence according to SEQ ID NO: 27, are combined with each other, and

(xii) Kp, a "p" number of K modules, namely 8 to 16 K modules, represented by the amino acid sequence according to SEQ ID NO: 23, are combined with each other.

More preferably,

(i) z in $(C)_m NR_z$ or $NR_z(C)_m$ is 1 and m is an integer of 8 to 48 (i.e. 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, or 48), z in $(C)_n NR_z$ or $NR_z(C)_m$ is 2 and m is an integer of 8 to 48 (i.e. 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, or 48), or z in $(C)_m NR_z$ or $NR_z(C)_m$ is 3 and m is an integer of 8 to 48 (i.e. 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, or 48),

(ii) z in $(AQ)_n NR_z$ or $NR_z(AQ)_n$ is 1 and n is an integer of 4 to 24 (i.e. 4, 5, 6, 7, 8, 9, 10, 11, 12, 13, 14, 15, 16, 17, 18, 19, 20, 21, 22, 23, or 24), z in $(AQ)_n NR_z$ or $NR_z(AQ)_n$ is 2 and n is an integer of 4 to 24 (i.e. 4, 5, 6, 7, 8, 9, 10, 11, 12, 13, 14, 15, 16, 17, 18, 19, 20, 21, 22, 23, or 24), or z in $(AQ)_n NR_z$ or $NR_z(AQ)_n$ is 3 and n is an integer of 4 to 24 (i.e. 4, 5, 6, 7, 8, 9, 10, 11, 12, 13, 14, 15, 16, 17, 18, 19, 20, 21, 22, 23, or 24),

(iii) z in $NR_z(QAQ)_o$ or $(QAQ)_o NR_z$ is 1 and o is an integer of 2 to 20 (i.e. 2, 3, 4, 5, 6, 7, 8, 9, 10, 11, 12, 13, 14, 15, 16, 17, 18, 19, or 20), z in $NR_z(QAQ)_o$ or $(QAQ)_o NR_z$ is 2 and o is an integer of 2 to 20 (i.e. 2, 3, 4, 5, 6, 7, 8, 9, 10, 11, 12, 13, 14, 15, 16, 17, 18, 19, or 20), or z in $NR_z(QAQ)_o$ or $(QAQ)_o NR_z$ is 3 and o is an integer of 2 to 20 (i.e. 2, 3, 4, 5, 6, 7, 8, 9, 10, 11, 12, 13, 14, 15, 16, 17, 18, 19, or 20),

wherein NR stands for a non-repetitive unit, preferably NR3 or NR4.

Most preferably, the silk polypeptide used in the method of the present invention is $C_{16}NR3$, $C_{32}NR3$, $C_{16}NR4$, $C_{32}NR4$, $(AQ)_{12}$, $(AQ)_{24}$, $(AQ)_{12}NR3$, $(AQ)_{24}NR3$, $(AQ)_{12}NR4$, $(AQ)_{24}NR4$, $C_{16}$, $C_{32}$, $Y_8$, $Y_{16}$, $X_8$, $X_{16}$, $K_8$, or $K_{16}$.

An ADF-3, ADF-4, MaSp I or MaSp II variant differs from the reference (wild-type) ADF-3 (SEQ ID NO: 1), ADF-4 (SEQ ID NO: 2), MaSp I (SEQ ID NO: 43) or MaSp II (SEQ ID NO: 44) polypeptide from which it is derived by up to 150 (up to 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, 100, 110, 120, 130, 140, or 150) amino acid changes in the amino acid sequence (i.e. substitutions, insertions, deletions, N-terminal truncations and/or C-terminal truncations). Such a variant can alternatively or additionally be characterised by a certain degree of sequence identity to the reference (wild-type) polypeptide from which it is derived. Thus, an ADF-3, ADF-4, MaSp I or MaSp II variant has a sequence identity of at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or even 99.9% to the respective reference (wild-type) ADF-3, ADF-4, MaSp I or MaSp II polypeptide. Preferably, the sequence identity is over a continuous stretch of at least 20, 25, 30, 35, 40, 50, 60, 70, 80, 90, 100, 120, 150, 180, 200, 250, 300, 350, 400, or more amino acids, preferably over the whole length of the respective reference (wild-type) ADF-3, ADF-4, MaSp I or MaSp II polypeptide.

It is particularly preferred that the sequence identity is at least 80% over the whole length, is at least 85% over the whole length, is at least 90% over the whole length, is at least 95% over the whole length, is at least 98% over the whole length, or is at least 99% over the whole length of the respective reference (wild-type) ADF-3, ADF-4, MaSp I or MaSp II polypeptide. It is further particularly preferred that the sequence identity is at least 80% over a continuous stretch of at least 20, 30, 50, 100, 150, 200, 250, or 300 amino acids, is at least 85% over a continuous stretch of at least 20, 30, 50, 100, 150, 200, 250, or 300 amino acids, is at least 90% over a continuous stretch of at least 20, 30, 50, 100, 150, 200, 250, or 300 amino acids, is at least 95% over a continuous stretch of at least 20, 30, 50, 100, 150, 200, 250, or 300 amino acids, is at least 98% over a continuous stretch of at least 20, 30, 50, 100, 150, 200, 250, or 300 amino acids, or is at least 99% over a continuous stretch of at least 20, 30, 50, 100, 150, 200, 250, or 300 amino acids of the respective reference (wild-type) ADF-3, ADF-4, MaSp I or MaSp II polypeptide.

A fragment (or deletion variant) of the ADF-3 (SEQ ID NO: 1) polypeptide has preferably a deletion of up to 1, 2, 3, 4, 5, 6, 7, 8, 9, 10, 15, 20, 25, 30, 35, 40, 45, 50, 55, 60, 65, 70, 75, 80, 85, 90, 95, 100, 120, 150, 170, 200, 220, 250, 270, 300, 320, 350, 370, 400, 420, 450, 470, 500, 520, 550, 570, 600, or 610 amino acids at its N-terminus and/or at its C-terminus. The deletion can also be internally.

A fragment (or deletion variant) of the ADF-4 (SEQ ID NO: 2) polypeptide has preferably a deletion of up to 1, 2, 3, 4, 5, 6, 7, 8, 9, 10, 15, 20, 25, 30, 35, 40, 45, 50, 55, 60, 65, 70, 75, 80, 85, 90, 95, 100, 120, 150, 170, 200, 220, 250, 270, 300, 320, 330, 340, 350, 360, 370, 380, or 390 amino acids at its N-terminus and/or at its C-terminus. The deletion can also be internally.

A fragment (or deletion variant) of the MaSp I (SEQ ID NO: 43) polypeptide has preferably a deletion of up to 1, 2, 3, 4, 5, 6, 7, 8, 9, 10, 15, 20, 25, 30, 35, 40, 45, 50, 55, 60, 65, 70, 75, 80, 85, 90, 95, 100, 150, 200, 250, 300, 350, 400, 450, 500, 550, 600, 620, 640, 660, 670, 680, or 690 amino acids at its N-terminus and/or at its C-terminus. The deletion can also be internally.

A fragment (or deletion variant) of the MaSp II (SEQ ID NO: 44) polypeptide has preferably a deletion of up to 1, 2, 3, 4, 5, 6, 7, 8, 9, 10, 15, 20, 25, 30, 35, 40, 45, 50, 55, 60, 65, 70, 75, 80, 85, 90, 95, 100, 150, 200, 250, 300, 350, 400, 450, 500, 520, 540, 560, or 570 amino acids at its N-terminus and/or at its C-terminus. The deletion can also be internally.

Additionally, the ADF-3, ADF-4, MaSp I or MaSp II variant or fragment is only regarded as an ADF-3, ADF-4, MaSp I or MaSp II variant or fragment within the context of the present invention, if the modifications with respect to the amino acid sequence on which the variant or fragment is based do not negatively affect the ability of a silk polypeptide to form a fibre. Preferably, it is still possible to draw a smooth and homogenous fibre from the silk polypeptide comprising the ADF-3, ADF-4, MaSp I or MaSp II variant or fragment at a speed of at least 0.1 cm/s, preferably 10 cm/s and more preferably 10 m/s. The skilled person can visually assess whether a fibre is still formed. The smooth and homogenous appearance of said fibre can be controlled using electronic-microscopy.

Preferably, the method is carried out at temperatures of between 5°C and 60°C, more preferably at temperatures of between 10°C and 50°C and most preferably, at temperatures of between 20°C and 40°C. Thus, the method can be carried out at a temperature of 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60°C.

Preferably, the method is carried out at a pressure of between 10 kPa and 1000 kPa, more preferably between 40 kPa and 500 kPa and most preferably between 50 kPa and 150 kPa. Thus, the method can be carried out at 10, 20, 30, 40, 50, 60, 70, 80, 90, 100, 150, 200, 250, 300, 350, 400, 450, 500, 550, 600, 650, 700, 750, 800, 950, 1000 kPa. It is particularly preferred that the method is carried out at standard conditions for temperature and pressure, i.e. at 20°C and 101.325 kPa.

In preferred embodiments of the present invention, the spinning solution provided in step (a) comprises more than one polymer, preferably 2, 3, or 4 polymers, i.e. a silk polypeptide and casein, zein or BSA. For example, the spinning solution provided in step (a) can comprise (i) a silk polypeptide and casein, (ii) a silk polypeptide and zein, (iii) a silk polypeptide and BSA, (iv) a silk polypeptide, zein, and casein, (v) a silk polypeptide, casein and BSA, (vi) a silk polypeptide, BSA, casein and zein. The polypeptides can be labeled, for example, with enzymatic labels, biotin, radioactive or fluorescent labels, e.g. fluorescein (FITC).

In other preferred embodiments of the present invention, the spinning solution provided in step (a) comprises more than one type of silk polypeptide. Preferably, the spinning solution provided in step (a) of the method of the present invention comprises 2, 3, 4, 5, 6, 7, 8, 9, or 10 different types of silk polypeptides, most preferably 2 different types of silk polypeptides. For example, the spinning solution can comprise dragline spider silk polypeptides, which differ from each other with respect to their amino acid sequence. The solution provided in the method of the present invention can also comprise dragline spider silk and flagelliform spider silk polypeptides which differ from each other with respect to their natural origin. The dragline spider silk polypeptide is from the major ampullate gland, while the flagelliform polypeptide is from the flagelliform gland.

The concentration of the silk polypeptide, and optionally BSA, zein or casein, is preferably in the range of 0.15 mg/ml to 500 mg/ml, more preferably in the range of 0.15 mg/ml to 99.9 mg/ml, in the range of 0.15 mg/ml to 90 mg/ml, in the range of 0.15 mg/ml to 80 mg/ml, in the range of 0.15 mg/ml to 70 mg/ml, in the range of 0.15 mg/ml to 60 mg/ml, or in the range of 0.15 mg/ml to 50 mg/ml, most preferably in the range of 0.5 mg/ml to 50 mg/ml, in the range of 0.5 mg/ml to 40 mg/ml, in the range of 0.5 mg/ml to 30 mg/ml or in the range of 1 mg/ml to 20 mg/ml. It is particularly preferred that the concentration of the polymer, e.g. silk polypeptide, BSA, zein or casein, is in the range of 0.15 mg/ml to 20 mg/ml.

Thus, for example, the concentration of the silk polypeptide, and optionally casein, zein or BSA, is (at least) 0.15 mg/ml, 0.2 mg/ml, 0.3 mg/ml, 0.4 mg/ml, 0.5 mg/ml, 0.6 mg/ml, 0.7 mg/ml, 0.8 mg/ml, 0.9 mg/ml, 1 mg/ml, 2 mg/ml, 3 mg/ml, 4 mg/ml, 5 mg/ml, 6 mg/ml, 7 mg/ml, 8 mg/ml, 9 mg/ml, 10 mg/ml, 11 mg/ml, 12 mg/ml, 13 mg/ml, 14 mg/ml, 15 mg/ml, 16 mg/ml, 17 mg/ml, 18 mg/ml, 19 mg/ml, 20 mg/ml, 21 mg/ml, 22 mg/ml, 23 mg/ml, 24 mg/ml, 25 mg/ml, 26 mg/ml, 27 mg/ml, 28 mg/ml, 29 mg/ml, 30 mg/ml, 31 mg/ml, 32 mg/ml, 33 mg/ml, 34 mg/ml, 35 mg/ml, 36 mg/ml, 37 mg/ml, 38 mg/ml, 39 mg/ml, 40 mg/ml, 41 mg/ml, 42 mg/ml, 43 mg/ml, 44 mg/ml, 45 mg/ml, 46 mg/ml, 47 mg/ml, 48 mg/ml, 49 mg/ml, 50 mg/ml, 55 mg/ml, 60 mg/ml, 65 mg/ml, 70 mg/ml, 75 mg/ml, 80 mg/ml, 85 mg/ml, 90 mg/ml, 95 mg/ml, 100 mg/ml, 110 mg/ml, 120 mg/ml, 130 mg/ml, 140 mg /ml, 150 mg/ml, 160 mg/ml, 170 mg/ml, 180 mg/ml, 190 mg/ml, 195 mg/ml, 200 mg/ml, 250 mg/ml, 300 mg/ml, or 350 mg/ml.

It should be noted that the method of the present invention requires only very low concentrations of silk polypeptides, beginning at concentrations as low as 0.15 mg/ml and, preferably, ending at concentrations as not higher as 90 mg/ml, more preferably as not higher as 50 mg/ml and most preferably as not higher as 20 mg/ml. This has the advantage that the shear viscosity of the spinning solution used in the method of the present invention is very low, which, in turn, improves the processability of said spinning solution.

The viscosity increasing compound is PAA, which increases the elongational viscosity of the spinning solution.

Preferably, the PAA has a molecular weight of at least 20 kDa, e.g. 25 kDa, 30 kDa, 40 kDa, 50 kDa, 60 kDa, 70 kDa, 80 kDa, 90 kDa, 100 kDa, 200 kDa, 300 kDa, 400 kDa, 500 kDa, 600 kDa, 700 kDa, 800 kDa, 900 kDa, 1 MDa, 1.5 MDa, 2 MDa, 2.5 MDa, 3 MDa, 3.5 MDa, 4 MDa, 4.5 MDa, or 5 MDa. It is preferred that the PAA has a molecular weight of between 20 kDa to 5 MDa or of between 50 kDa to 4 MDa, more preferably of between 100 kDa to 2 MDa or of between 100 kDa to 1 MDa.

More preferably, the PAA has a molecular weight of at least 500 kDa, e.g. 550 kDa, 600 kDa, 650 kDa, 700 kDa, 750 kDa, 800 kDa, 850 kDa, 900 kDa, 950 kDa, 1 MDa, 1.5 MDa, 2 MDa, 2.5 MDa, 3 MDa, 3.5 MDa, 4 MDa, 4.5 MDa, or 5 MDa. It is more preferred that the linear or branched polymer has a molecular weight of between 500 kDa to 5 MDa or of between 900 kDa to 5 MDa, most preferably of between 1 MDa to 4 MDa or of between 1 MDa to 3 MDa.

The concentration of the PAA is preferably in the range of 0.1 wt%/vol to 5 wt%/vol, more preferably in the range of 0.5 wt%/vol to 4 wt%/vol, and most preferably in the range of 1 wt%/vol to 3 wt%/vol.

Thus, for example, the concentration of the PAA is 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5 4.6, 4.7, 4.8, 4.9, or 5 wt%/vol.

The inventors of the present invention surprisingly found that already this low concentration of PAA is sufficient for spinning fibres with the method provided herein. The low concentration of PAA increases on the one hand side the elongational viscosity of the solution, but on the other hand side does not substantially increase the shear viscosity of the solution so that the generation of fibres is improved. Thus, the spinning solution used in the method of the present invention is mainly composed of a silk polypeptide i.e. is relatively pure. Further, due to the sparely use of compounds or substances in addition to the silk polypeptide, the method of the present invention is cost efficient and ecologically friendly.

The ratio by weight% of the silk polypeptide to PAA is preferably between 0.1 to 5, 0.2 to 5, 0.3 to 5, or 0.4 to 5, more preferably between 0.4 to 2, most preferable between 0.8 to 1.5. Thus, in preferred embodiments, wherein the silk polypeptide is present in the solution in a range of 1 to 20 mg/ml the amount of PAA is chosen in such that the ratio by weight% of the silk polypeptide to PAA is preferably between 0.1 to 10, more preferably between 0.2 to 5, more preferably between 0.4 to 2, most preferable between 0.8 to 1.5.

For example, by adding high molecular weight PAA (5 MDa, 1% wt/vol), the Ohnesorge number of an $C_{16}$ spinning solution can be increased sufficiently in order to allow improved fibre formation at the air/liquid interface.

The solvent can be any solution in which the silk polypeptide such as the spider silk polypeptide, and the PAA are soluble. It is preferred that the solvent is selected from the group consisting of a polar solvent, preferably water, an aqueous buffer, an alcohol, preferably isopropanol, Hexafluoroisopropanol, glycerol ethanol (EtOH), propanol, butanol, octanol, or acetone; a nonpolar solvent, preferably hexane, dodecane or oil, other organic solvents, preferably ethylacetate; and mixtures thereof. Thus, the solvent can also be a mixture of a polar solvent, e.g. water, and a non-polar solvent, e.g. hexane. In a preferred embodiment, the pH of the solvent is between 4 and 10.

It should be noted that due to the combinatory use of very low concentrations of silk polypeptides, and very low concentrations of PAA, fibre formation can be facilitated with the method of the present invention.

In addition, due to the combinatory use of very low concentrations of silk polypeptides, and very low concentrations of PAA, said solution does not have a highly viscous consistency in contrast to other spinning solutions of the prior art. Thereby, the processability of the spinning solution can be improved.

Preferably, the drawing of the fibre is initiated by contacting an air/liquid or liquid/liquid interface of the solution with a drawing tool and withdrawing the drawing tool from said interface. The term "air" as used herein preferably means the mixture of gases naturally occurring in the earth atmosphere. In the context of a liquid/liquid interface, wherein one of the liquids is the spinning solution it is preferred that the other liquid is poorly miscible with the spinning solution. Accordingly, if the solvent of the spinning solution is a polar solvent the other liquid is preferably a non-polar. Alternatively, if the solvent of the spinning solution is a non-polar solvent preferably the other liquid is polar. A liquid/liquid interface may also be formed at least temporarily when two miscible liquids are brought into contact, e.g. in a tube, wherein the one liquid stream is enclosing a stream of the second liquid. Without wishing to be bound by any theory it is believed that the initial contacting of the interface with a drawing tool, e.g. a pipette tip, provides the polymers in the spinning solution with an attachment point. The withdrawing of the pipette leads to an orientation of the polymers in the solvent with its head or tail towards the drawing tool, whereby the polymers align side-by-side and increasingly associate with each other. Upon further withdrawing the polymers transition from an ordered solution or liquid crystal type state to a semi solid or solid state. The term "drawing" is used in this context not only refer to the initiation of this alignment process but also to refer to continued withdrawing of aligned or partially aligned polymer molecules from the spinning solution. During this state of, preferably continuous drawing, the drawing tool no longer contacts the spinning solution but the "drawing" of further polymer molecules from the solution into a "detached solution", i.e. a solution comprising aligned polymers no longer in contact with the body of the spinning solution. Thus, in the context of the present invention, the term "drawing" means drawing as a process of fibre formation, which can also include stretching or hardening. Furthermore, drawing the fibre from the solution is not a process restricted to pulling a fibre from a detached solution with a drawing tool like, e.g. a glass needle as demonstrated in example 1. The process can also comprise any appropriate automated system, for example, a pipe through which the solution is driven in a replenishing flow, with a nozzle at the end where the spinning solution extrudes and is being guided away by a drawing tool, thereby continuously drawing the fibre from the extruded spinning solution. The drawing tool can be any kind of instrument with a tip appropriate for drawing a fibre and of an appropriate material, e.g. capable of providing an attachment point to the polymers in the spinning solution. Examples include, but are not limited to pipette tips, needles or thin bars made of plastic, metal or glass. The speed of fibre-drawing is not limited, as long as it is high enough for drawing a thin fluid filament, which exists long enough to allow evaporation of the solvent and the formation of a fibre, preferably a thin, light and long fibre. However, in a preferred embodiment drawing is carried out by touching the solution with a drawing tool and the drawing of a fibre from the surface. In operation, i.e. when the initial contact with the surface has been made and a fibre has been drawn from the surface the term drawing also refers to the withdrawing of the fibre from the solution, preferably at a constant speed. The ideal speed depends on the viscosity of the spinning solution, i.e. on type and concentration of the polymers used, and has to be determined by experiment. Preferably, the drawing is carried out at speeds of at least 0.1 cm/s, preferably between 0.1 cm/s and 15 m/s, between 1 cm/s and 5 m/s, between 3 cm/s and 1 m/s, between 5 cm/s and 50 cm/s, between 5 cm/s and 15 cm/s, and more preferably at 10 cm/s. For example, the drawing is carried out at speeds of at least 0.1, 0.5, 1, 2, 3, 4, 5, 6, 7, 8, 9, 10, 11, 12, 13, 14, 15, 20, 30, 40, 50, 60, 70, 80, 90 cm/s, 1, 1.5, 2, 2.5, 3, 3.5, 4, 4.5, 5, 6, 7, 8, 9, 10, 11, 12, 13, 14, or 15 m/s.

In the process of the invention it is also envisioned that the spinning solution is extruded to form a fibre. The term "extrusion" in this context means the application of pressure to the spinning solution to force it through an opening, e.g. a nozzle. In the art of polymer technology "extrusion" processes are often used to form fibres from molten thermoplasts, i.e. the entire extruded material solidifies after it has left the opening. In the context of the present invention the extruded spinning solution does not solidify entirely. Rather the dissolved polymers that are comprised in the extruded spinning solution associate to form a fibre, which is typically smaller in diameter than the opening through which the spinning solution is extruded, while the remaining solvent is separated from the fibre thus formed. In a preferred embodiment the fibre initially formed is attached to a fibre recovery device, e.g. a cylinder, spool or bobbin, onto which the fibre is continuously wound. Depending on the relative speed of the, e.g. cylinder, with respect to the speed of fibre formation during extrusion, there may also be a pulling force exerted onto the extruded fibre, i.e. the fibre in some embodiments may be considered to be "drawn" from to extruded solution.

To further improve the spinnability of the spinning solution, the shear viscosity of artificial spinning dopes can be increased simultaneously to the elongational viscosity to an amount not detrimental to fluid processing by, for example, adding Newtonian viscosity enhancers. Spinnability thereby means the suitability of a spinning solution for fibre drawing.

Thus, in one embodiment of the invention, the spinning solution further comprises a compound that increases Newtonian viscosity, preferably monosaccharides, e.g. glucose, galactose, or fructose; disaccharides, e.g. lactose, or sucrose; polysaccharides, e.g. glycogen; or polyols, e.g. inositiol, sorbitol, glycerol, or polyglycols; or mixtures thereof.

Preferably, the spinning solution further comprises a particulate matter dispersed therein. Upon fibre formation, this particulate matter is embedded in the fibre in a relatively uniform manner. The particulate matter is not limited in its composition as long as it is suitable for being incorporated into a fibre. It is preferred that the particulate matter is selected from the group consisting of beads, electricity conducting metals (including gold, copper), crystallites, protein/protein-complexes, enzymes (e.g. green fluorescent protein (GFP) or β-galactosidase) and cells (e.g. human osteoblast cells or human tendon precursor cells or any pluripotent cells or stem cells). Preferably, the cells are selected from enzyme or hormone secreting cells, e.g. Langerhans islet cells. Preferred crystallites are dyes which are not soluble in water, fluorescent dyes (e.g. Sudan red) or azo dyes (e.g. Nile red).

The concentration of the particulate matter in the spinning solution is not limited. Preferably, the particulate matter is present in the spinning solution in an amount in the range of 0.01 wt%/vol to 10 wt%/vol, preferably 0.5 wt%/vol to 1 wt%/vol. Thus, the particulate matter can be present in the spinning solution in an amount of 0.01, 0.05, 0.1, 0.2, 0.3, 0.4, 0.5, 0.6, 0.7, 0.8, 0.9, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10.0 wt%/vol.

In a preferred embodiment of the invention, the spinning solution further comprises a compound that increases Newtonian viscosity, e.g. polyglycols, or a particulate matter, e.g. beads. In another preferred embodiment, the spinning solution further comprises both a compound that increases Newtonian viscosity, e.g. polyglycols, and a particulate matter, e.g. beads.

The fibre can be treated after formation by curing in a curing solution or cross-linking. Thus, it is preferred that the method of the present invention comprises subsequent to step (b) a step (c) of curing the fibre in a curing solution or cross-linking the fibre.

Cross-linking can comprise linking other compounds to the fibre or linking identical or different fibres to each other, whereby, for example, multi-fibre-structures such as multi-layer fibres or core-shell structures can be produced.

The term "cross-linking" as used herein refers to a process of chemically joining two molecules by a covalent bond. Cross-linking or coupling reagents contain reactive ends to specific functional groups (primary amines, sulfhydryl, etc.) on polypeptides or on other molecules. Preferably, the cross-linking or coupling reagent used in the method of the present invention is 1-ethyl-3-(3-dimethylaminopropyl)-carbodiimide hydrochloride (EDC) or Glutaraldehyde.

In another embodiment, the fibre is coiled while continuously drawn. This allows the production and storage of long fibres.

In a second aspect the present invention relates to a spinning solution for carrying out the method of the present invention, comprising, essentially consisting of or consisting of (i) a silk polypeptide (ii) PAA and (iii) a solvent.

It is further particularly preferred that the concentration of the silk polypeptide, is at least 0.15 mg/ml and not more than 60 mg/ml, more preferably at least 0.15 mg/ml and not more than 50 mg/ml, and most preferably at least 0.15 mg/ml and not more than 20 mg/ml.

It is also particularly preferred that the concentration of the PAA is in the range of 0.1 wt%/vol to 5 wt%/vol, more preferably in the range of 0.5 wt%/vol to 4 wt%/vol, and most preferably in the range of 1 wt%/vol to 3 wt%/vol.

It is particularly more preferred that the concentration of the silk polypeptide, is at least 0.15 mg/ml and not more than 60 mg/ml, more preferably at least 0.15 mg/ml and not more than 50 mg/ml, and most preferably at least 0.15 mg/ml and not more than 20 mg/ml and that the concentration of the PAA is in the range of 0.1 wt%/vol to 5 wt%/vol, more preferably in the range of 0.5 wt%/vol to 4 wt%/vol, and most preferably in the range of 1 wt%/vol to 3 wt%/vol.

All other components of the spinning solution mentioned above in the context of the description of the method of the invention can also be comprised in the spinning solution according to this aspect of the invention. This similarly applies to all preferred and particularly preferred embodiments of the various components mentioned above.

In a third aspect, the present invention provides a fibre obtainable by the method of the first aspect.

In a fourth aspect, the present invention provides products comprising the fibre of the second aspect, such as

    (i) a thread, or
    (ii) a woven fabric or a non-woven fabric.

In a further aspect, the present invention provides the use of a fibre or thread comprising one or more cells to assay one or more cellular functions and/or to maintain said cells. Preferably, the cellular function is assayed prior, during and/or after exposing the one or more cells to a test compound or to a stimulus, preferably to a mechanical, thermal and/or electric stimulus. Cell-containing fibres could also serve as scaffold material for tissue engineering and the growth or artificial organs.

The present invention improves the spinnability of a polymer solution, comprising a silk polypeptide and a PAA, allowing for the production of a whole range of novel materials. The present invention is particularly suited for the incorporation of living and non-living biological material, because the process can be carried out at appropriate conditions regarding temperature, pressure, solvent and chemicals used.

Since generally all proteins can be incorporated into fibres in this manner, any enzymatic activity can in principle be incorporated into such fibres. Perhaps more importantly, cells can be embedded in fibres as well, as has been shown with the incorporation of colloidal micron-size particles into fibres. Since the conditions favour cell viability, applications in bioengineering and tissue culture are possible. Cell-containing fibres could serve as scaffold material for tissue engineering and the growth of artificial organs. Also, encapsulating living cells allows subjecting those cells to differing environmental conditions, by pulling them through a bath with reactants, for instance. With such approaches, new tools for cell biology may be accessible. This can be used for serial and also high-throughput manipulation of cells on a string. One possibility is drawing a fibre with cells embedded from a spinning solution comprising a bioactive compound, and screening the cells as they come out of the solution. Thereby, the position on the string constitutes an index for the cells, providing new approaches in single-cell high-throughput screening assays. In addition, applications in wound healing are possible. For example, fibres containing cells secreting wound healing factors could be incorporated into or supplement bandages or xerogels, particularly in a wet wound healing context. Conceivable are also uses in the production of skin grafts, replacement ligaments or surgical mesh. Also, fibres produced according to the invention can be used in a wide range of other commercial products, for example rope, netting, clothing fabric, construction material, tent fabric, backpacks and in many others which have strength, elasticity and low weight as desired characteristics.

## BRIEF DESCRIPTION OF THE FIGURES

**Fig.: 1**    shows fibres drawn from a spinning solution comprising 5 mg/ml FITC-$C_{16}$ and 0.5 wt%/vol PAA. A plain glass needle is pulled away from an aliquot of spinning solution (A), resulting in the formation of a thin fluid filament (B). (C) Fibres drawn at a speed of at least 10 cm/s appear smooth and homogenous. (D) Fibres drawn at a speed below the appropriate speed threshold. Droplets of liquid are still coating the fluid filament and remain after fibre deposition on a clean glass microscopy slide.

Fig.: 2    shows fibres drawn from a spinning solution comprising approximately 107/ml Polystyrene beads (1 $\mu$m diameter) dispersed in 5 mg/ml $C_{16}$ and 0.5 wt%/vol PAA. The beads are incorporated in the fibres and distributed fairly homogenously.

Fig.: 3    shows Atomic Force Microscopy Image of a fibre drawn from a spinning solution comprising Polystyrene beads (1 $\mu$m diameter) dispersed in 5 mg/ml $C_{16}$ and 0.5 wt%/vol PAA. The beads are covered with fibre material and are tightly connected to the fibre.

Fig.: 4    shows zeine fibre drawn from a spinning solution comprising 300 mg/ml zeine and 0.5% wt/vol PAA in 63.75% ethanol. The zeine fibre exhibited a smooth surface.

EXAMPLES

The following examples are for illustrative purposes only and do not limit the invention described above in any way.

**Example 1: Principle of the drawing process**

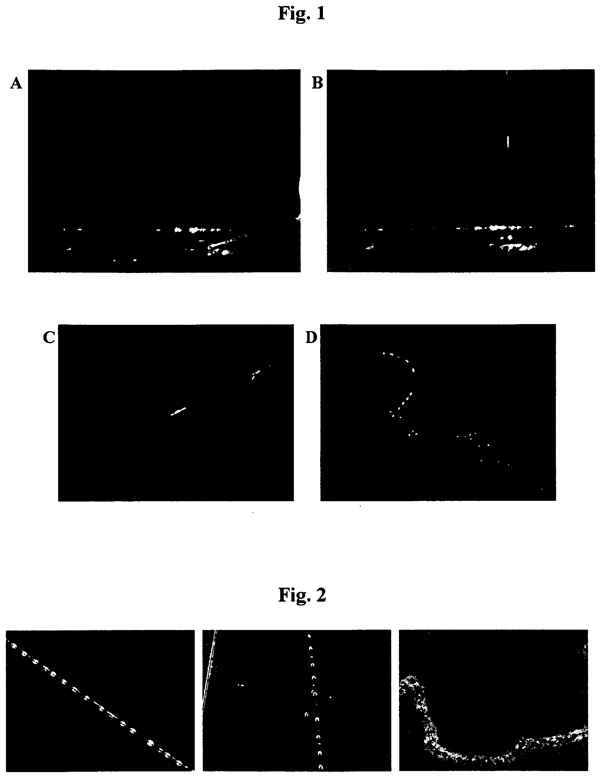

**Fig. 1 A** shows an aliquot of spinning solution consisting of $C_{16}$ as the constituent polymer, PAA as the elongational viscosity enhancing polymer, and water as the solvent on a glass microscopy slide, in contact with a plain glass needle as the drawing tool. Pulling the needle, which is in contact with the spinning solution, away from the solution produces a thin fluid filament. This filament, if drawn quickly enough, exists long enough to allow evaporation of the solvent water and the formation of a thin and light fibre (**Fig. 1 B**). The fibres contain protein, which can be shown by IR spectroscopy and by fluorescence microscopy when labelled protein is used.

**Example 2: Fibres from a low concentration spider silk protein**

100 $\mu$l spinning solution (10 mg/ml FITC-$C_{16}$, 1.0 wt%/vol PAA) were placed on a glass microscopy slide. A plain glass needle (5 cm long and 1 mm in diameter) was brought into contact with the spinning solution and then pulled away from the microscopy slide by hand at variable speeds. This process resulted in a fibre being pulled from the spinning solution, the quality of the fibre depended on the speed used. At or above a pulling speed of 10 cm/s, the fibres appeared smooth and homogenous (**Fig. 1 C**). When pulling at speeds below the threshold, droplets of liquid were still coating the fluid filament and remained after fibre deposition on a clean glass microscopy slide (**Fig. 1 D**). Good fibre formation occurred at a pulling speed of 10 cm/s and above.

**Example 3: Fibres pulled by a falling steel bead**

100 $\mu$l spinning solution (10 mg/ml $C_{16}$, 1.0 wt%/vol PAA) were placed next to the rim of glass microscopy slide. A steel bead of 1 mm diameter was placed between rim and a spinning solution, touching the spinning solution. While the microscopy slide was being held by hand, it was tilted slightly to cause the bead falling off the rim. Surprisingly, the falling bead pulled a fibre from the spinning solution not unlike the needle from Example 1. The process of the bead falling and producing a fibre can be compared to a falling spider producing a fibre, although obviously the reservoir of spinning dope is at different ends of the fibre.

**Example 4: Embedding beads in fibres**

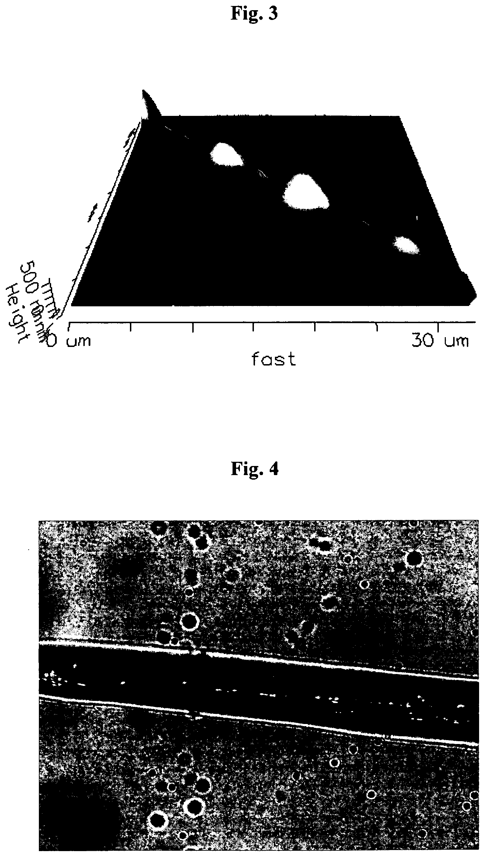

Approximately 10[7]/ml Dynabeads (Polystyrene or PS beads) of 1 $\mu$m diameter were dispersed in 100 $\mu$l spinning solution (10 mg/ml $C_{16}$, 1.0 wt%/vol PAA). A plain glass needle (5 cm long and 1 mm in diameter) was brought into contact with the spinning solution and then pulled away from the microscopy slide by hand at a speed of approximately 10 cm/s. This process resulted in a fibre containing PS beads being pulled from the spinning solution. The beads were embedded in the fibre and distributed fairly homogenously (**Fig. 2**). Atomic Force Microscopy showed that the beads were covered with fibre material and were tightly connected to the fibre (**Fig. 3**).

Since this experiment can be carried out at conditions appropriate for living cells in terms of temperature, pressure and solvent, it demonstrates the possibility of incorporating cells instead of the PS beads used, which had a diameter similar to those of many cells, for instance fibroblasts.

**Reference example 5: Fibres from zeine**

Zeine is a storage protein from corn and has been used as a material for decades. It is available in reasonable purity in bulk quantities at low cost (Tsai 1979). 300 mg/ml zeine in 85% ethanol were mixed with 2% wt/vol PAA, with a final PAA concentration of 0.5% wt/vol. 100 $\mu$l of this spinning solution were placed on a glass microscopy slide. A plain glass needle (5 cm long and 1 mm in diameter) was brought into contact with the spinning solution and then pulled away from the microscopy slide by hand at a speed of approximately 10 cm/s. This process resulted in a zeine fibre being pulled from the spinning solution (**Fig. 4**). Such zeine fibres were insoluble in water and had a smooth surface. They appeared to have good mechanical stability, but were rather brittle and not ductile. Zeine fibres could be turned more ductile in a formaldehyde bath.

**Example 6: Incorporating of crystallites into fibres**

Dyes (Sudan Red (Sigma 20161-8) or Nile Red (Fluka 72485) were added as a fine powder to the spinning solution (10 mg/ml C$_{16}$, 1.0 wt%/vol PAA) in suspension. Both dyes have been incorporated into the fibres. Both dyes are hydrophobic and adhere strongly to the hydrophobic spider silk proteins.

**Example 7: Incorporating of enzymes into fibres**

Fluorescent proteins such as Green Fluorescent Protein (GFP) have been incorporated into the fibres (10 mg/ml C$_{16}$, 1.0 wt%/vol PAA). After incorporation bright fluorescence has been observed. This shows that the folding of the protein (GFP) in the fibers and therefore the protein itself is still intact.
β-galactosidase has been incorporated into the fibers (10 mg/ml C$_{16}$, 1.0 wt%/vol PAA). By enzymatic hydrolization o-nitrophenyl-β-Dgalactopyranosid (ONPG) is hydrolyzed to the yellow dye o-nitrophenol. The concentration of o-nitrophenol is determined to quantify the activity of β-galactosidase in the fibre. The incorporation of enzymes can be used for any applications where enzymes that are large enough to be trapped in the fibre while substances can diffuse in and out are needed.

**Claims**

1. A method of spinning a fibre from a spinning solution comprising the steps of:

   (a) providing a spinning solution comprising (i) a silk polypeptide, (ii) polyacrylamide (PAA), and (iii) a solvent; and
   (b) drawing a fibre from the spinning solution or a combination of extruding and drawing a fibre from the spinning solution, whereby a fibre is formed.

2. The method of claim 1, wherein the silk polypeptide comprises at least two identical repetitive units each comprising at least one consensus sequence selected from the group consisting of:

   i) GPGXX (SEQ ID NO: 3), wherein X is any amino acid, preferably in each case independently selected from A, S, G, Y, P, N, and Q;
   ii) GGX, wherein X is any amino acid, preferably in each case independently selected from Y, P, R, S, A, T, N and Q; and
   iii) A$_x$, wherein x is an integer from 5 to 10.

3. The method of claims 1 to 2, wherein the drawing of the fibre is initiated by contacting an air/liquid interface of the solution with a drawing tool and withdrawing the drawing tool from said interface.

4. The method of claims 1 to 3, wherein the spinning solution further comprises a compound that increase Newtonian viscosity, preferably monosaccharides, polysaccharides, or polyols.

5. The method of claims 1 to 4, wherein the spinning solution further comprises a particulate matter dispersed therein, wherein the particulate matter is preferably selected from the group consisting of cells, more preferably cells selected from the group consisting of enzyme secreting cells, hormone secreting cells, stem cells and neuronal cells, beads, electricity conducting metals, crystallites, proteins/proteincomplexes, enzymes and growth factors.

6. The method of claim 5, wherein the particulate matter is present in the spinning solution in an amount in the range of 0.01 wt%/vol to 10 wt%/vol, preferably 0.5 wt%/vol to 1 wt%/vol.

7. A spinning solution for carrying out the method of claims 1 to 6, comprising

   (i) a silk polypeptide,
   (ii) polyacrylamide (PAA), and
   (iii) a solvent.

8. A fibre obtainable by a process of claims 1 to 6.

9. A thread comprising a fibre of claim 8.

10. A woven or non-woven fabric comprising a fibre of claim 8.

11. Use of a fibre of claim 8 or a thread of claim 9 comprising one or more cells, to assay one or more cellular functions and/or to maintain said cells, wherein the cellular function is preferably assayed prior, during and/or after exposing the one or more cells to a test compound or to a stimulus, more preferably to a mechanical, thermal and/or electric stimulus.

**Patentansprüche**

1. Verfahren zum Spinnen einer Faser aus einer Spinnlösung, umfassend die Schritte:

   (a) Bereitstellen einer Spinnlösung, umfassend (i) ein Seidenpolypeptid, (ii) Polyacrylamid (PAA) und (iii) ein Lösungsmittel; und
   b) Ziehen einer Faser aus der Spinnlösung oder einer Kombination aus Extrudieren und Ziehen einer Faser aus der Spinnlösung, wodurch eine Faser gebildet wird.

2. Verfahren nach Anspruch 1, wobei das Seidenpolypeptid mindestens zwei identische repetitive Einheiten umfasst, wobei jede mindestens eine Konsensussequenz umfasst, welche aus der Gruppe bestehend aus

> i) GPGXX (SEQ ID NO: 3), wobei X jede Aminosäure, vorzugsweise in jedem Fall unabhängig ausgewählt aus A, S, G, Y, P, N und Q, ist,
> ii) GGX, wobei X jede Aminosäure, vorzugsweise in jedem Fall unabhängig ausgewählt aus Y, P, R, S, A, T, N, und Q, ist,
> iii) $A_x$, wobei x jede ganze Zahl von 5 bis 10 ist,

ausgewählt ist.

3. Verfahren nach den Ansprüchen 1 bis 2, wobei das Ziehen der Faser durch Kontaktieren einer Luft-/Flüssigkeitszwischenphase der Lösung mit einem Zugwerkzeug und dem Zurückziehen des Zugwerkzeugs von der Zwischenphase eingeleitet wird.

4. Verfahren nach den Ansprüchen 1 bis 3, wobei die Spinnlösung ferner eine Verbindung, die die Newtonsche Viskosität erhöht, vorzugsweise Monosaccharide, Polysaccharide oder Polyole, umfasst.

5. Verfahren nach den Ansprüchen 1 bis 4, wobei die Spinnlösung ferner ein partikuläres Mittel, welches darin dispergiert ist, umfasst, wobei das partikuläre Mittel vorzugsweise aus der Gruppe bestehend aus Zellen, bevorzugter Zellen ausgewählt aus der Gruppe bestehend aus Enzym-sekretierenden Zellen, Hormon-sekretierenden Zellen, Stammzellen und neuronalen Zellen, Kügelchen, elektrisch-leitfähigen Metallen, Kristalliten, Proteinen/Proteinkomplexen, Enzymen und Wachstumsfaktoren ausgewählt ist.

6. Verfahren nach Anspruch 5, wobei das partikuläre Mittel in der Spinnlösung in einer Menge in einem Bereich von 0,01 Gew.-%/V bis 10 Gew.-%/V, vorzugsweise 0,5 Gew.-%/V bis 1 Gew.-%/V vorhanden ist.

7. Spinnlösung zur Durchführung des Verfahrens nach den Ansprüchen 1 bis 6, umfassend

> (i) ein Seidenpolypeptid,
> (ii) Polyacrylamid (PAA), und
> (iii) ein Lösungsmittel.

8. Faser, erhältlich nach einem Verfahren nach den Ansprüchen 1 bis 6.

9. Faden, umfassend eine Faser nach Anspruch 8.

10. Gewebter oder nicht-gewebter Stoff, umfassend eine Faser nach Anspruch 8.

11. Verwendung einer Faser nach Anspruch 8 oder eines Fadens nach Anspruch 9, die/der eine Zelle oder mehrere Zellen umfasst, zum Testen von einer Zellfunktion oder mehrerer Zellfunktionen und/oder zum Aufrechterhalten der Zellen, wobei die zelluläre Funktion vorzugsweise vor, während und/oder nach der Exposition der einen Zelle oder der mehreren Zellen zu einer Testverbindung oder zu einem Stimulus, bevorzugter zu einem mechanischen, thermischen, thermischen und/oder elektrischen Stimulus, getestet wird.

**Revendications**

1. Méthode de filage d'une fibre à partir d'une solution de filage comprenant les étapes :

> (a) de fourniture d'une solution de filage comprenant (i) un polypeptide de soie, (ii) du polyacrylamide (PAA), et (iii) un solvant ; et
> (b) d'étirage d'une fibre à partir de la solution de filage ou une combinaison d'extrusion et d'étirage d'une fibre à partir de la solution de filage, par quoi une fibre est formée.

2. Méthode selon la revendication 1, dans laquelle le polypeptide de soie comprend au moins deux motifs de répétition identiques comprenant chacun au moins une séquence consensus sélectionnée dans le groupe constitué par :

> i) GPGXX (SEQ ID NO : 3), dans laquelle X est un acide aminé quelconque, préférablement dans chaque cas indépendamment sélectionné parmi A, S, G, Y, P, N, et Q ;
> ii) GGX, dans laquelle X est un acide aminé quelconque, préférablement dans chaque cas indépendamment sélectionné parmi Y, P, R, S, A, T, N et Q ; et
> iii) $A_x$, dans laquelle x est un nombre entier de 5 à 10.

3. Méthode selon les revendications 1 à 2, dans laquelle l'étirage de la fibre est initié par la mise en contact d'une interface air/liquide de la solution avec un outil d'étirage et le retrait de l'outil d'étirage de ladite interface.

4. Méthode selon les revendications 1 à 3, dans laquelle la solution de filage comprend en outre un composé qui augmente la viscosité newtonienne, préférablement des monosaccharides, des polysaccharides, ou des polyols.

5. Méthode selon les revendications 1 à 4, dans laquelle la solution de filage comprend en outre une matière particulaire dispersée dans celle-ci, dans laquelle la matière particulaire est préférablement sélectionnée dans le groupe constitué par des cellules, plus préférablement des cellules sélectionnées dans le groupe constitué par des cellules sécrétant des enzymes, des cellules sécrétant des hormones, des cellules souches et des cellules neuronales, des perles, des métaux conducteurs d'électricité, des cristallites, des complexes de protéine(s), des enzymes et des facteurs de croissance.

6. Méthode selon la revendication 5, dans laquelle la matière particulaire est présente dans la solution de filage dans une quantité dans la plage de 0,01 % poids/volume à 10 % poids/volume, préférablement de 0,5 % poids/volume à 1 % poids/volume.

7. Solution de filage pour réaliser la méthode selon les revendications 1 à 6, comprenant

  (i) un polypeptide de soie,
  (ii) du polyacrylamide (PAA), et
  (iii) un solvant.

8. Fibre pouvant être obtenue par un procédé selon les revendications 1 à 6.

9. Fil comprenant une fibre selon la revendication 8.

10. Tissu tissé ou non tissé comprenant une fibre selon la revendication 8.

11. Utilisation d'une fibre selon la revendication 8 ou d'un fil selon la revendication 9 comprenant une ou plusieurs cellules, pour tester une ou plusieurs fonctions cellulaires et/ou pour entretenir lesdites cellules, dans laquelle la fonction cellulaire est préférablement testée avant, pendant et/ou après l'exposition des une ou plusieurs cellules à un composé d'essai ou à un stimulus, plus préférablement à un stimulus mécanique, thermique et/ou électrique.

Fig. 1

Fig. 2

**Fig. 3**

**Fig. 4**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 03057727 A
- WO 08155304 A
- WO 2006008163 A
- WO 2008155304 A
- WO 2007025719 A

### Non-patent literature cited in the description

- **GOSLINE et al.** *J. Exp. Biol.,* 1999, vol. 202, 3295
- **SCHEIBEL.** *Microb. Cell. Fact.,* 2004, vol. 1, 14
- **HUEMMERICH et al.** *Curr. Biol.,* 2004, vol. 22, 2070-4
- **RAMMENSEE et al.** *PNAS,* 2008, vol. 105, 6590-6595
- **EXLER et al.** *Angewandte Chemie-International Edition,* 2007, vol. 19, 3559-3562
- A multilingual glossary of biotechnological terms: (IUPAC Recommendations). Helvetica Chimica Acta. 1995
- **ELVIN et al.** *Nature,* 2005, vol. 473, 999-1002